# EUROPEAN PATENT SPECIFICATION

(11) **EP 3 966 224 B1**
(45) Date of publication and mention of the grant of the patent: **28.01.2026**
(21) Application number: 20802944.7
(22) Date of filing: 06.05.2020
(51) Int. Cl.: C07K 1/16, G01N 33/483, C07K 1/36, G01N 33/68

(54) **METHODS FOR THE FRACTIONATION OF PROTEINS**
VERFAHREN ZUR FRAKTIONIERUNG VON PROTEINEN
PROCÉDÉS DE FRACTIONNEMENT DE PROTÉINES

(30) Priority: 06.05.2019 AU 2019901536
(43) Date of publication of application: 16.03.2022
(73) Proprietor: The University of Sydney, Sydney NSW 2006 (AU)
(72) Inventor: LARANCE, Mark, Sydney, New South Wales 2006 (AU)
(74) Representative: Zacco Sweden AB
(86) International application number: PCT/AU2020/050444
(87) International publication number: WO 2020/223761

(56) References cited:
- WO-A1-91/15223
- WO-A1-97/31943
- CN-A- 104 292 305
- GB-A- 1 214 891
- US-A- 3 953 290
- US-A1- 2006 188 956
- HARNEY DYLAN J. ET AL: "Small-protein Enrichment Assay Enables the Rapid, Unbiased Analysis of Over 100 Low Abundance Factors from Human Plasma", MOLECULAR & CELLULAR PROTEOMICS, vol. 18, no. 9, 15 July 2019 (2019-07-15), US, pages 1899 - 1915, XP93063989, ISSN: 1535-9476, Retrieved from the Internet <URL:https://www.ncbi.nlm.nih.gov/pmc/articles/PMC6731089/pdf/zjw1899.pdf> DOI: 10.1074/mcp.TIR119.001562
- PICKART L ET AL: "RESPONSES OF RAT AND CHICK CHONDROCYTES AND RAT HEPATOMA CELLS TO PLASMA FRACTIONS WITH INSULIN-LIKE AND GROWTH-PROMOTING ACTIVITIES", BIOCHIMICA ET BIOPHYSICA ACTA,, vol. 632, 1 January 1980 (1980-01-01), pages 112 - 120, XP001345226
- PLAUT K ET AL: "Evaluation of interference by insulin-like growth factor I (IGF-I) binding proteins in a radioimmunoassay for IGF-I in serum from dairy cows", DOMESTIC ANIMAL ENDOCRINOLOGY, ELSEVIER, AMSTERDAM, NL, vol. 8, no. 3, 1 July 1991 (1991-07-01), pages 393 - 405, XP023546024, ISSN: 0739-7240, [retrieved on 19910701], DOI: 10.1016/0739-7240(91)90007-7
- LIU C ET AL: "Identification of relaxin-3/INSL7 as an endogenous ligand for the orphan G-protein-coupled receptor GPCR135", JOURNAL OF BIOLOGICAL CHEMISTRY, AMERICAN SOCIETY FOR BIOCHEMISTRY AND MOLECULAR BIOLOGY, US, vol. 278, no. 50, 12 December 2003 (2003-12-12), pages 50754 - 50764, XP002319829, ISSN: 0021-9258, DOI: 10.1074/JBC.M308995200
- LIN LIN ET AL: "High throughput and accurate serum proteome profiling by integrated sample preparation technology and single-run data independent mass spectrometry analysis", JOURNAL OF PROTEOMICS, ELSEVIER, AMSTERDAM, NL, vol. 174, 24 December 2017 (2017-12-24), pages 9 - 16, XP085412679, ISSN: 1874-3919, DOI: 10.1016/J.JPROT.2017.12.014
- HARNEY DYLAN J. ET AL: "Small-protein Enrichment Assay Enables the Rapid, Unbiased Analysis of Over 100 Low Abundance Factors from Human Plasma", vol. 18, no. 9, 15 July 2019 (2019-07-15), US, pages 1899 - 1915, XP093063989, ISSN: 1535-9476, Retrieved from the Internet <URL:https://www.ncbi.nlm.nih.gov/pmc/articles/PMC6731089/pdf/zjw1899.pdf> DOI: 10.1074/mcp.TIR119.001562
- POSNER B I, GUYDA H J, CORVOL M T, RAPPAPORT R, HARLEY C, GOLDSTEIN S: "Partial Purification, Characterization, and Assay of a Slightly Acidic Insulin-Like Peptide (ILAs) from Human Plasma", JOURNAL OF CLINICAL ENDOCRINOLOGY AND METABOLISM, vol. 47, no. 6, 1 December 1978 (1978-12-01), US , pages 1240 - 1250, XP009531041, ISSN: 0021-972X, DOI: 10.1210/jcem-47-6-1240
- BALKIS EDDHIF, JUSTIN LANGE, NADIA GUIGNARD, YANN BATONNEA, JONATHAN CLARHAUT, SÉBASTIEN PAPOT,CLAUDE GEFFROY-RODIER, PAULINE POIN: "Study of a novel agent for TCA precipitated proteins washing - comprehensive insights into the role of ethanol/HCl on molten globule state by multi- spectroscopic analyses", JOURNAL OF PROTEOMICS, vol. 173, 2018, pages 77 - 88, XP055760195
- KEMNA, E.H.J.M. ET AL.: "Hepcidin: from discovery to differential diagnosis", HAEMATOLOGICA, vol. 93, 2008, pages 90 - 97, XP002502643, DOI: 10.3324/HAEMATOL.11705
- GUNDRY R L, WHITE M Y, MURRAY C I, KANE L A, FU Q, STANLEY B A, VAN EYK J E: "Preparation of proteins and peptides for mass spectrometry analysis in a bottom-up proteomics workflow.", CURRENT PROTOCOLS IN MOLECULAR BIOLOGY,20091001JOHN WILEY & SONS, INC, US, no. Suppl. 90, 1 October 2009 (2009-10-01), US , pages 10.25 - 10.25-23, XP002753520, ISSN: 1934-3639, DOI: 10.1002/0471142727.mb1025s88
- BAO QUOC TRAN, CELINE HERNANDEZ, PATRICE WARIDEL, ALEXANDRA POTTS, JACHEN BARBLAN, FREDERIQUE LISACEK, MANFREDO QUADRONI: "Addressing trypsin bias in large scale (phospho)proteome analysis by size exclusion chromatography and secondary digestion of large post-trypsin peptides", JOURNAL OF PROTEOME RESEARCH, vol. 10, 2011, pages 800 - 811, XP055760199

## Description

### Technical Field

The present invention relates generally to the field of protein analysis. More specifically, the present invention provides methods for the fractionation of low molecular weight proteins from mixed protein populations. The methods described herein find particular application in the specific detection and quantitation of low abundance/low molecular weight proteins in complex biological samples.

### Background

Low abundance small-protein hormones such as insulin and hepcidin play pivotal roles in metabolic homeostasis, and their functional disruption contributes to diverse disease states. Insulin typically occurs in human plasma at <10 ng/mL. Genetic and acquired aberrations in insulin function are major contributing factors in type 2 diabetes and obesity, and high plasma insulin levels are indicative of insulin resistance, a precursor to diabetes. Insulin resistance can predispose an individual to a range of other disorders from hypertension to cancer. Rare insulin-producing tumours can cause dangerous hypoglycaemia, due to the dependence of the brain on blood glucose.

Hepcidin-25 is a low abundance 25-amino acid liver-derived hormone that controls systemic iron homeostasis by inhibiting iron absorption in the small intestine and inhibiting iron release from the liver. Hepcidin-25 is often over-produced during inflammation, resulting in anaemia. Levels of hepcidin-25 in patient samples are being increasingly used as biomarkers for a range of diseases associated with iron metabolism, including anaemia associated with chronic kidney disease, and hereditary hemochromatosis, which causes a toxic build-up of iron in major organs.

The specific detection and quantitation of low abundance small-protein hormones in human biological samples plays a key role in clinical diagnosis, and a global view of the low molecular weight plasma proteome has the potential to identify novel biomarkers and therapeutic molecules, and advance the field of precision medicine. Currently, analysis of low abundance small-protein hormones is often performed with assays employing antibodies such as ELISAs or radioimmunoassays (RIAs). While these assays can be fast and sensitive, they are expensive, only quantify one small-protein hormone at a time, and preclude global analyses and hypothesis-free discovery. In addition, antibody-based assays are not always specific enough to distinguish between active chains of hormones and either their isoforms, or degradation products.

Untargeted mass spectrometry-based proteomic analysis could overcome these issues, but a major problem is the large dynamic range of blood plasma protein abundance covering ~13 orders of magnitude. For example, many protein hormones such as insulin exist in plasma at abundances <10 ng/mL, whereas albumin is ~45 mg/mL. The approach taken by most groups to deeply characterize the plasma proteome and detect these factors either employs depletion of the top 5-60 most abundant proteins using antibody-based columns, peptide-level fractionation such as high-pH reverse phase separations, or the sequential combination of both techniques, prior to downstream MS analysis. While this approach is effective, it is expensive, throughput is low as a very large amount of instrument time (days to weeks) is required to acquire the data for each sample, and sample volume available for subsequent analysis is limited. Additionally, biases may be introduced during immunodepletion due to cross-reactivity of antibodies, and the binding of small-protein hormones to carrier proteins.

Previous work on global analysis of small-protein hormones in plasma has tested a variety of procedures to overcome this problem. One approach to enrich for low abundance small-protein hormones has been solvent- or organic acid-based precipitation. However, a major problem with this technique is the co-precipitation of small-protein hormones with highly abundant high molecular weight carrier proteins, for example, >99% of circulating insulin-like growth factor 1 (IGF-1) is bound to insulin-like growth factor-binding proteins (IGFBPs). Variations of this technique involving the addition of a supported liquid membrane (SLM) extraction step, in which a carrier-mediated coupled transport system utilises ionic carriers to transport short peptides between two aqueous phases through an organic liquid membrane, have had limited success in terms of the number of peptides recovered from plasma samples. Again, associations of peptides with larger molecular weight proteins have presented a barrier to the enrichment process, and levels of salts in plasma have also hindered the success of these techniques. The existence in biological samples of small-protein hormones bound to high molecular weight proteins has also hindered the success of enrichment procedures employing ultrafiltration and/or size exclusion chromatography.

Hydrochloric acid (HCl) is a relatively poor protein precipitant when compared to halogenated organic acids (trifluoroacetic acid (TFA) or trichloroacetic acid (TCA)) frequently used in proteomics studies. Halogenated organic acids typically facilitate precipitation by several mechanisms including depletion of the hydration shell around proteins to increase hydrophobic interactions and disrupting ionic interactions through pH changes. However, this is not the case for HCl treatment, which only mediates pH-based dissociation of ionic interactions. Likewise, ethanol is also a poor protein precipitant (although it is commonly used for albumin precipitation) when compared with acetonitrile (ACN). The low ability of HCl to precipitate high molecular weight proteins has precluded its consideration as a precipitant for enrichment for small-protein hormones for global analyses. Without adequate removal of high molecular weight proteins, the large number of different tryptic peptides that can be produced from each >10kDa protein disfavours the detection of the relatively few tryptic peptides that can be produced from small-protein hormones.

Many previous protocols have omitted disulfide bond reduction and alkylation, potentially introducing either intramolecular, or intermolecular crosslinked peptides for downstream analysis by tandem mass spectrometry. The resulting complex fragmentation spectra are largely not compatible with standard database search methodologies and require specialized analysis.

Unbiased and sensitive detection and quantitation of low abundance small-protein hormones such as insulin in biological samples with a high dynamic range of protein abundance remains an unmet need. A need also exists in precision medicine for an inexpensive method of creating an unbiased global view of active small-protein hormones within a short timescale which can enable individual characterization of disease at the protein level. No method has yet detected peptides from a wide variety of clinically-relevant, active, small-protein hormones such as insulin, IGF-1, hepcidin and RANTES. The detection of peptides derived from the active chains of each hormone is key and previous untargeted methods largely detect pro-peptide derived sequences, for example, the C-peptide from insulin, but not either the beta or alpha chains.

CN104292305A relates to extraction of a polypeptide comprising 19 amino acids using HCl-ethanol and then size exclusion chromatography.

In contrast to genomics, the field of proteomics has several challenges to overcome before the proteins in a biological sample can be characterized at a global level, including limitations and variability of sample size, the dynamic range problem, multiplicity of isoforms and variations in concentration due to time, disease status and/or drug perturbations. In particular, a substantial need exists for a solution to the dynamic range problem, and a method for enrichment for small-protein hormones that will facilitate unbiased and sensitive detection and simultaneous measurement in downstream analysis and enable the discovery of novel hormones for use as biomarkers and therapeutic targets.

### Summary of the Invention

The present invention meets at least one of the needs mentioned above by providing methods for dissociating proteins from binding partners and/or specific isolation and/or subsequent fractionation of low molecular weight proteins, for example, proteins within the molecular weight range of 15 kDa to 0.5 kDa. The methods of the present invention may include concomitant removal of high molecular weight proteins and/or low molecular weight degradation products. The methods of the present invention may enable accurate detection and and/or quantitation in downstream analysis, which may occur within a short timescale.

The present invention relates to the following embodiments:
In a first aspect, the present invention provides a method for separating proteins with a molecular weight under 15 kDa from plasma or serum, the method comprising the steps of:
contacting the plasma or serum in an extraction buffer comprising hydrochloric acid and ethanol to provide an extraction mixture; and
separating the proteins with a molecular weight under 15 kDa from the extraction mixture by ultra high pressure liquid chromatography (UHPLC) and size exclusion chromatography;
further comprising the steps of:
   (i) disulfide bond reduction of the proteins separated from the extraction mixture;
   (ii) alkylation of reduced sulfhydryl groups in the proteins after step (i); and
   (iii) contacting the proteins with a protease after step (ii);
prior to then analyzing the proteins by mass spectrometry after step (iii).

In one embodiment of the first aspect, the proteins have a molecular weight under 14 kDa, under 13 kDa, under 12 kDa, under 11 kDa, under 10 kDa, under 9 kDa, under 8 kDa, under 7 kDa, under 6 kDa, under 5 kDa, under 4 kDa, under 3 kDa, under 2 kDa, under 1 kDa, above 1 kDa or above 0.5 kDa.

In one embodiment of the first aspect, hydrochloric acid is present in the extraction buffer at a concentration of between 0.05M and 0.5M, between 0.1M and 0.4M, between 0.2M and 0.3M, or between 0.2M and 0.25M.

In one embodiment of the first aspect, ethanol is present in the extraction buffer at a concentration of under 95% (v/v), under 90% (v/v), under 80% (v/v), under 70% (v/v), under 60% (v/v), or under 50% (v/v).

In one embodiment of the first aspect, the is present in the extraction mixture at a volume that is under 40%, under 30%, under 20%, under 10% or under 5% of the extraction buffer volume within the extraction mixture.

In one embodiment of the first aspect, the size exclusion chromatography is denaturing size exclusion chromatography.

In one embodiment of the first aspect, the comprises the use of solid phase extraction and/or supported liquid extraction to remove a lipid from said reaction mixture.

In one embodiment of the first aspect, the protease used in step (iii) is lysC and/or trypsin.

In one embodiment of the first aspect, the disulfide bond reduction is performed with DTT.

In one embodiment of the first aspect, the proteins are fractionated using high pH reverse phase chromatography following contacting the proteins with a protease, and prior to analyzing the proteins by mass spectrometry.

In one embodiment of the first aspect, the mass spectrometry is performed using a liquid chromatography-tandem mass spectrometer.

In one embodiment of the first aspect, analysing the proteins by mass spectrometry comprises an ionization technique selected from electrospray ionization and matrix-assisted laser desorption-ionization.

In one embodiment of the first aspect, analysing the proteins by mass spectrometry comprises a data acquisition method selected from the group consisting of data-dependent acquisition, data-independent acquisition, selected reaction monitoring and parallel reaction monitoring.

In one embodiment of the first aspect, the mass spectrometry is used to quantitate a single protein from the plasma or serum.

In one embodiment of the first aspect, the proteins comprise peptide hormones.

In one embodiment of the first aspect the proteins comprise or consist of insulin and/or hepicidin.

In one embodiment of the first aspect, the proteins with a molecular weight under 15kDa are present in the plasma or serume at a concentration of less than 1mg/mL, less than 900 µg/mL, less than 800 µg/mL, less than 700 µg/mL, less than 600 µg/mL, less than 500 µg/mL, less than 400 µg/mL, less than 300 µg/mL, less than 200 µg/mL, less than 100 µg/mL, less than 1 µg/mL, less than 900ng/mL, less than 800ng/mL, less than 700ng/mL, less than 600ng/mL, less than 500ng/mL, less than 400ng/mL, less than 300ng/mL, less than 200ng/mL, less than 100ng/mL, less than 1ng/mL, less than 900 pg/mL, less than 800 pg/mL, less than 700 pg/mL, less than 600 pg/mL, less than 500 pg/mL, less than 400 pg/mL, less than 300 pg/mL, less than 200 pg/mL, less than 100 pg/mL, less than 1 pg/mL, less than 900fg/mL, less than 800fg/mL, less than 700fg/mL, less than 600fg/mL, less than 500fg/mL, less than 400fg/mL, less than 300fg/mL, less than 200fg/mL, or less than 100fg/mL.

### Definitions

Certain terms are used herein which shall have the meanings set forth as follows.

As used in this application, the singular form "a", "an" and "the" include plural references unless the context clearly dictates otherwise. For example, the term "protein" also includes a plurality of proteins unless otherwise stated.

As used herein, the term "comprising" means "including". Variations of the word "comprising", such as "comprise" and "comprises" have correspondingly varied meanings. Thus, for example, a composition "comprising" may consist exclusively of or may include one or more additional components, for example, an extraction buffer "comprising" hydrochloric acid and ethanol may consist exclusively of hydrochloric acid and ethanol or may include one or more additional components, for example, water.

As used herein, the term "plurality" means more than one. In certain specific aspects or embodiments, a plurality may mean 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or more, and any numerical value derivable therein, and any range derivable therein.

As used herein, the term "between" when used in reference to a range of numerical values encompasses the numerical values at each endpoint of the range. For example, hydrochloric acid with a concentration of between 0.2M and 0.3M is inclusive of hydrochloric acid with a concentration 0.2M and hydrochloric acid with a concentration of 0.3M.

As used herein, the terms "protein" and "peptide" each refer to a polymer made up of amino acids linked together by peptide bonds and are used interchangeably. For the purposes of the present invention a "peptide" may constitute a full-length protein or a portion of a full-length protein.

As used herein, the term "solid phase extraction" refers to any method of sample preparation in which one or more compounds dissolved or suspended in a liquid mixture are separated from other component/s of said liquid mixture by sorption to a solid.

As used herein, the term "supported liquid extraction" refers to any method of sample preparation in which one or more compounds dissolved or suspended in a liquid mixture are separated from other component/s of said liquid mixture by sorption of the liquid mixture to an inert solid and selective removal of component/s of the mixture from said inert solid using one or more solvents.

### Abbreviations

The following abbreviations are used herein and throughout the specification:
*¹⁵N*: nitrogen-15 isotope
*µ*g: microgram/s
*µL*: microlitre/s
*µm*: micrometre/s
*Å*: angstrom
*ACN:* acetonitrile
*ACTH:* adrenocorticotropic hormone
*BMI:* body mass index
*CID:* collision-induced dissociation
*cm:* centimetre/s
*C-terminus:* carboxyl-terminus
CV: coefficient of variability
*C5ORF46*: chromosome 5 open reading frame 46
*DDA:* data-dependent acquisition
*DIA:* data-independent acquisition
*DTT:* dithiothreitol
*ECD:* electron capture dissociation
*EDTA:* ethylenediaminetetraacetic acid
*ELISA:* enzyme-linked immunosorbent assay
*ESI:* electrospray ionization
*FDR:* false discovery rate
*FFM:* fat free mass
g: gravitational force
*GIR:* glucose infusion rate
*GLP-1:* glucagon-like peptide 1
h: hour/s
*H₂SO₄*: sulfuric acid
*H₃PO₄*: phosphoric acid
*HCD:* higher energy collisional dissociation
*HCl:* hydrochloric acid
*HNI₃*: nitric acid
*HPLC:* high pressure liquid chromatography
*IF*: intermittent fasting
*IGF-1:* insulin-like growth factor 1
*IGF-2:* insulin-like growth factor 2
*IGFBP*: insulin-like growth factor binding protein
*IL-36gamma:* interleukin 36 gamma
*iRT:* indexed retention time
*kDa:* kilodalton/s
*kV*: kilovolts
*LC-MS*/*MS*: liquid chromatography-tandem mass spectrometry
*M*: molar
*min:* minute/s
*mL*: millilitre/s
*mm:* millimetre/s
*MRM:* multiple reaction monitoring
*ms:* millisecond/s
*m*/*z:* mass-to-charge ratio
*ng*: nanogram/s
*nL*: nanolitre/s
*NCE:* normalized collision energy
*nm:* nanometre/s
*nM:* nanomolar
*NMR:* nuclear magnetic resonance
*MS:* mass spectrum
*MS*/*MS*: tandem mass spectrometry
*NET:* neutrophil extracellular trap
*PaxDB:* Protein Abundance Database
*PCR:* polymerase chain reaction
*ppb:* parts per billion
*ppm:* parts per million
*PRM:* parallel reaction monitoring
*RIA*: radioimmunoassay
*RANTES:* Regulated upon Activation, Normal T cell Expressed, and Secreted
*rpm*: revolutions per minute
*RRA*: radioreceptor assay
*s*: seconds
*SDF-1:* stromal cell-derived factor 1
*SEC:* size exclusion chromatography
*SERPINA1*: the gene encoding alpha-1 antitrypsin, a serine protease inhibitor
*SLM:* supported liquid membrane
*SPAAT:* a short peptide from alpha-1 antitrypsin
*sPHEA:* small-protein hormone enrichment assay
*SRM:* selected reaction monitoring
*TEAB:* triethylammonium bicarbonate
*TCA:* trichloroacetic acid
*TCEP:* tris-(2-carboxyethyl) phosphine
*TFA:* trifluoroacetic acid
*Th:* theoretical fragment ion mass spectra
*THPP:* tris-(3-hydroxypropyl) phosphine
*UHPLC:* ultra high pressure liquid chromatography
*UV:* ultraviolet
*UVPD:* ultraviolet photodissociation

### Brief Description of the Figures

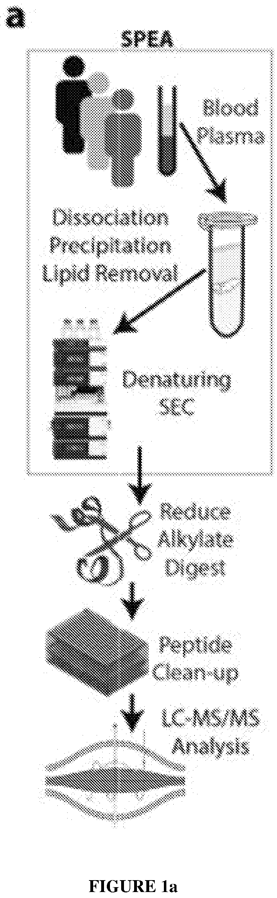

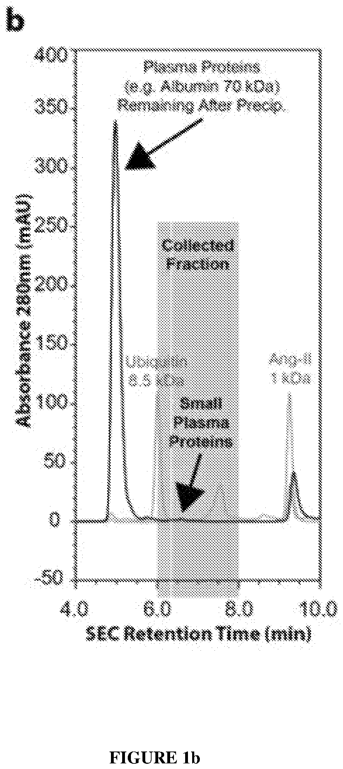

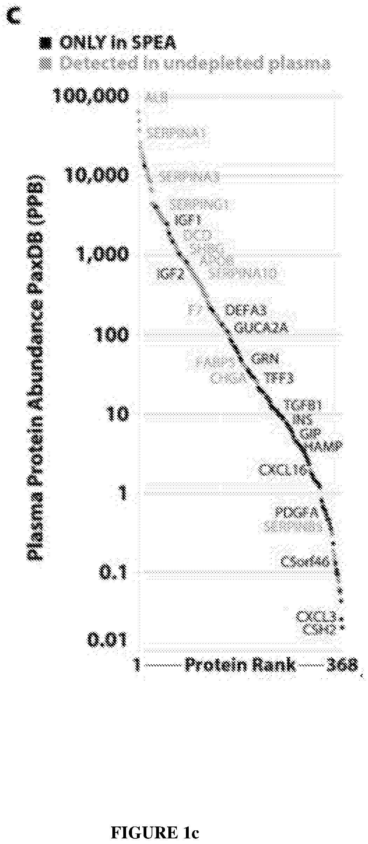

Embodiments of the present invention will now be described, by way of example only, with reference to the accompanying Figures 1-6 as set out below:
**Figure 1****: An overview of the sPHEA:** **Figure 1a** provides a schematic of a non-limiting example of a workflow for one embodiment of the present invention, referred to herein as the small-protein hormone enrichment assay (sPHEA). **Figure 1b** provides a non-limiting representative UV absorbance chromatogram of denaturing SEC separations for a human plasma sample (black) and two molecular weight standards (grey). A combination of protein-protein dissociation and protein removal using HCl and ethanol prior to denaturing high-resolution UHPLC-based SEC analysis enabled 25 minute molecular weight-based separations that were optimized for proteins <10 kDa, but also >2 kDa to avoid small degradation products. The absorbance profile highlighted the large amount of proteins >10 kDa remaining in the extract after the dissociation-precipitation procedure and the comparatively low levels of proteins below 10 kDa. The grey highlighted zone between 6-8 minutes was collected as one fraction for subsequent trypsin digestion and LC-MS/MS analysis. **Figure 1c** provides a ranked abundance plot of plasma proteins detected in either a fractionated deep proteome from raw undepleted human plasma (from Geyer *et al.,* 2016) (grey), or a fractionated deep proteome from the sPHEA (black). Protein abundance rank is derived from all previously detected plasma proteins from proteomics experiments in PaxDB.

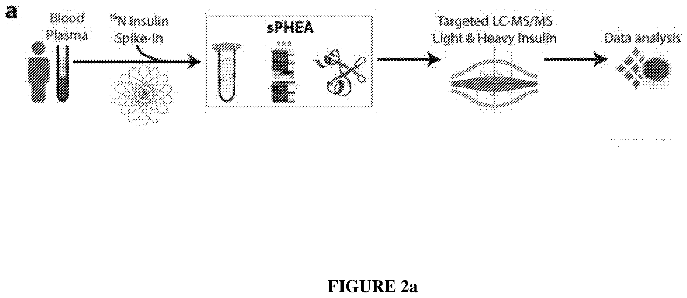

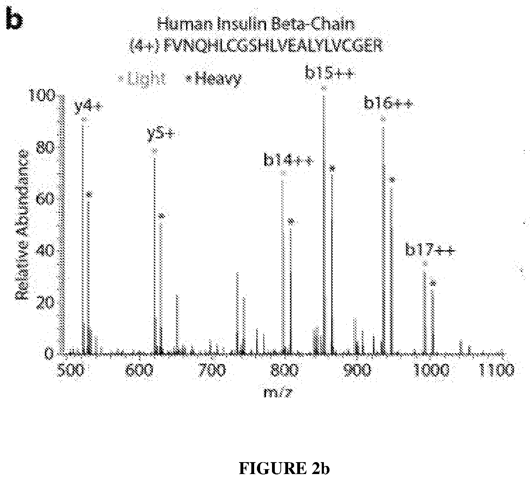

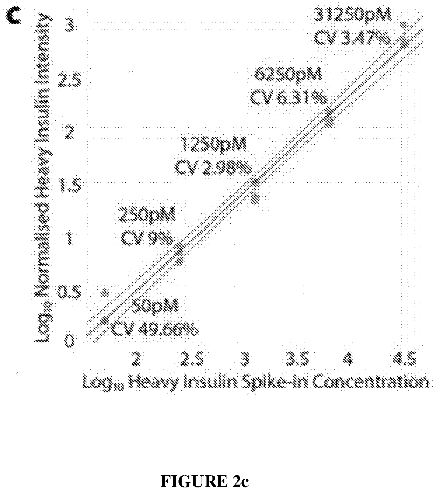

**Figure 2****: Absolute quantitation of insulin using heavy isotope labelling:** **Figure 2a** provides a schematic of a non-limiting workflow for (¹⁵N) heavy-insulin human plasma spike-in experiments. Using a stable-isotope (¹⁵N) labelled intact human insulin, a standard curve was established using spike-in of the heavy analogue to raw plasma prior to the start of the sPHEA workflow. A targeted PRM-based LC-MS/MS analysis method was established which combined quantitation of the light and heavy insulin beta chain peptide fragments in the same MS/MS (PRM) spectrum. **Figure 2b** provides a non-limiting representative MS/MS (PRM) spectrum of the targeted tryptic peptide derived from the human insulin beta-chain, which was carbamidomethylated at both cysteine residues. These data are generated from co-fragmentation under identical settings for both the heavy and light forms of the peptide with 6 unique fragment ions labelled. Fragment ions derived from the light (endogenous) insulin are marked with grey asterisks and fragment ions derived from the heavy (exogenous) insulin are marked with black asterisks. **Figure 2c** provides a non-limiting representative standard curve for the (¹⁵N) heavy-insulin in human plasma (n=3). The peptide chosen for PRM analysis was unique to the beta-chain of insulin. For quantitation, the intensities of multiple y and b fragment ions were used to generate a total intensity value for either the light or heavy insulin. The standard curve generated covered a concentration range spanning 3 orders of magnitude and showed excellent linearity and low CV values even at lower concentrations.

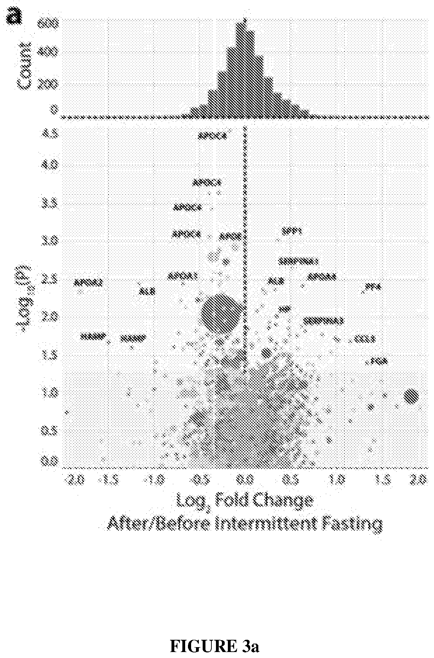

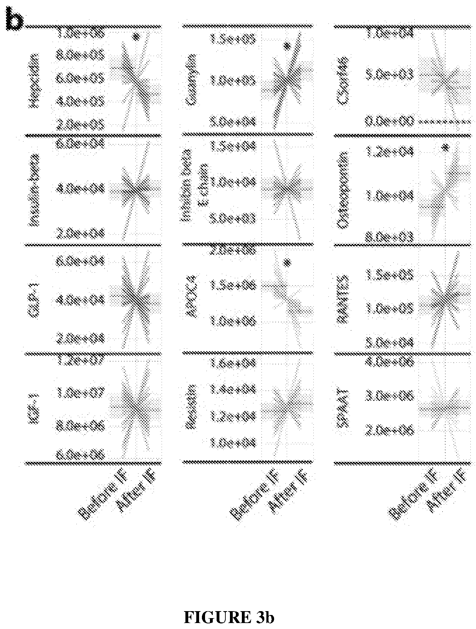

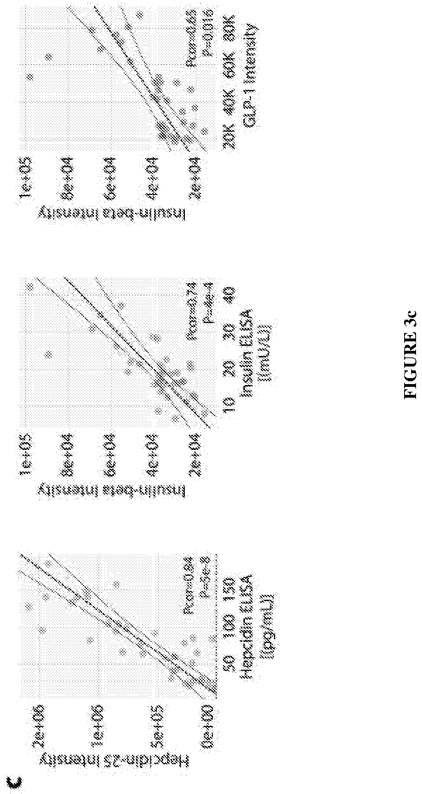

**Figure 3****: sPHEA analysis of a human intermittent fasting clinical trial.** **Figure 3** provides, by way of exemplification only, data obtained using the sPHEA during a clinical trial which examined the effects of intermittent fasting (IF) in humans (n=20), with blood plasma sampling before and after the 8-week IF intervention. **Figure 3a** provides a volcano plot for all the peptide precursors detected, with the log₂ fold change plotted on the x-axis versus the -log10(P) of a paired test statistic on the y-axis. The darker shaded region highlights non-significant p-values (>0.05). The size of the circles represents the abundance of the precursor peptide with larger being more abundant. Precursor peptides derived from the same gene share the same shade of grey. **Figure 3b** provides adjusted intensity plots for selected peptides of interest, where each line represents an individual patient. The grey line and surrounding ribbon represent the mean and the 95% confidence interval of the mean, respectively. Peptides that had a significant p-value (P<0.05) are marked with an asterisk. **Figure 3c** provides a correlation analysis across all patient samples of either the hepcidin-25 peptide MS intensity versus the hepcidin ELISA abundance values (left), the insulin beta-chain peptide MS intensity versus the insulin RIA abundance values (centre), or the insulin beta-chain peptide MS intensity versus the GLP-1 peptide MS intensity (right). The Pearson correlation and the associated Benjamini-Hochberg corrected p-value are shown inside each plot. The dashed lines represent the fitted model and its 95% confidence interval.

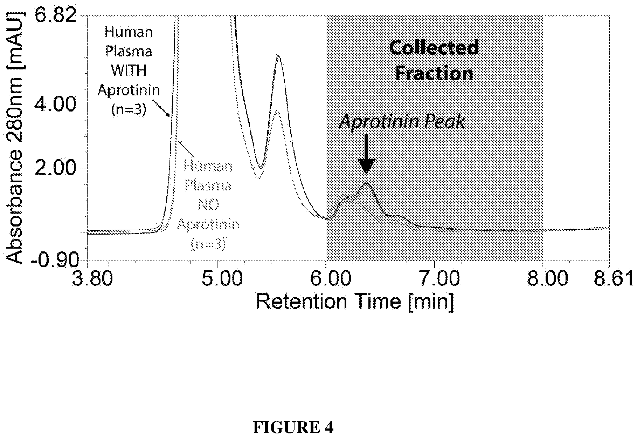

**Figure 4****: sPHEA analysis of plasma containing the protease inhibitor aprotinin.** **Figure 4** provides, by way of exemplification only, data obtained using the sPHEA method to process human plasma samples containing the protease inhibitor aprotinin, which is itself a protein and is commonly used to preserve hormones in blood plasma samples (n=3). Shown is a non-limiting representative UV absorbance chromatogram of denaturing SEC separations for human plasma samples containing aprotinin (black lines), or human plasma not containing aprotinin (grey lines). The absorbance profile highlighted that the amount of aprotinin present in the samples was small enough to elute within the normal SPEA fraction collection window (grey highlighted zone between 6-8 minutes).

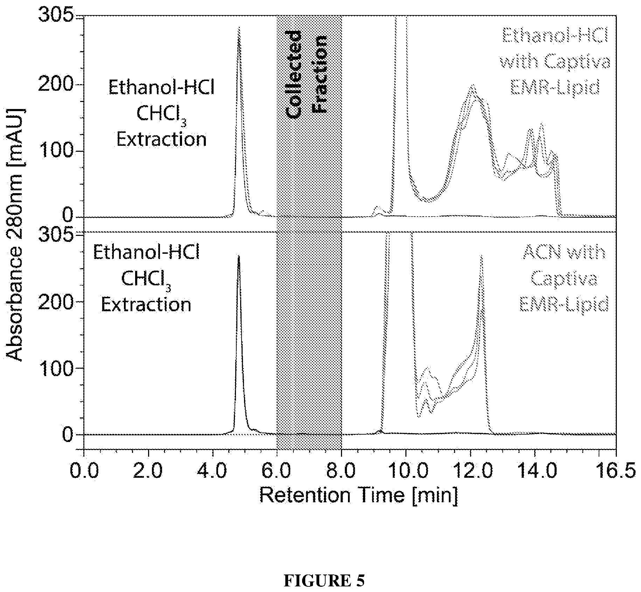

**Figure 5****: Testing high throughput sPHEA analysis of plasma using solid phase extraction for lipid removal instead of chloroform.** **Figure 5** provides, by way of exemplification only, data obtained using plasma that has had lipid removed (prior to SEC separation) either by manual chloroform (CHCl₃) extraction in tubes, or by 96-well plate solid phase extraction (Agilent Captiva EMR Lipid removal resin) (n=3). **Figure 5** **top panel** provides a non-limiting representative UV absorbance chromatogram of denaturing SEC separations for human plasma samples extracted using the ethanol-HCl buffer and chloroform in tubes for lipid removal (black lines), or human plasma samples extracted using the ethanol-HCl buffer and then lipid removal by 96-well plate solid phase extraction (Agilent Captiva EMR Lipid removal resin) (grey lines). The chloroform and 96-well lipid removal methods, when used with prior plasma treatment with ethanol-HCl provide similar protein/peptide recovery. **Figure 5** **bottom panel** provides a non-limiting representative UV absorbance chromatogram of denaturing SEC separations for human plasma samples extracted using either the ethanol-HCl buffer and chloroform in tubes for lipid removal (black lines), or human plasma samples extracted using acetonitrile and then lipid removal by 96-well plate solid phase extraction (Agilent Captiva EMR Lipid removal resin) (grey lines). Acetonitrile is the recommended extraction solvent for use with the Agilent Captiva EMR Lipid removal resin. However, the use of acetonitrile provides >90% reduction in protein/peptide recovery and should not be used for sPHEA analysis.

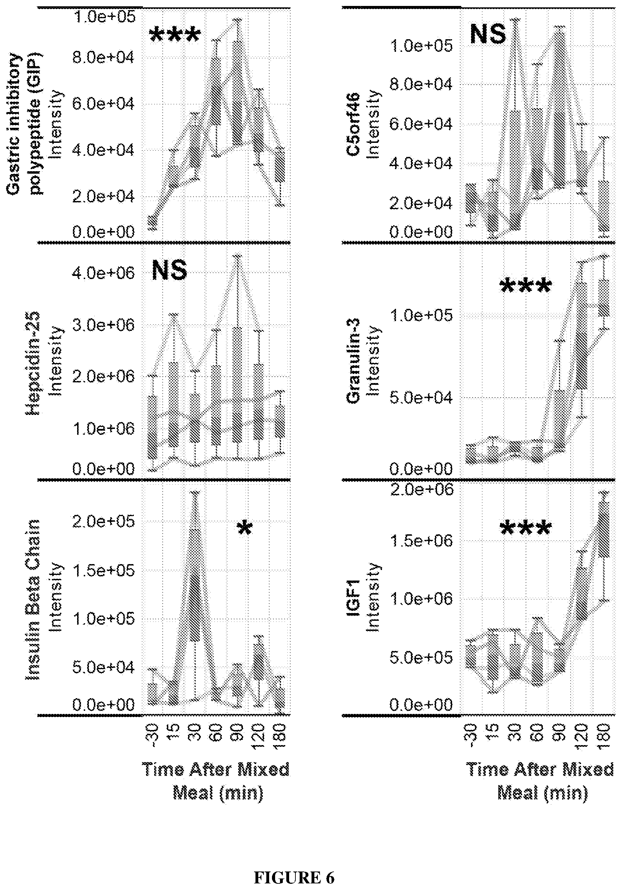

**Figure 6****: High throughput sPHEA analysis of a human mixed meal test.** **Figure 6** provides, by way of exemplification only, data obtained using the sPHEA method adapted for high throughput using 96-well plate solid phase extraction (Agilent Captiva EMR Lipid resin) on a clinical trial that examined the effects of food consumption over time in humans (n=4). Blood plasma (containing aprotinin to preserve hormones) was sampled before food was consumed in participants fasted for 10 hours and then at various timepoints after consumption of a meal. Line plots are shown for six example hormones detected, with time after the meal plotted on the x-axis versus the relative intensity on the y-axis. Each grey line shows the hormone abundance for one participant. Boxplots show the median and quartiles with the whiskers indicating 1.5 times the interquartile range. Hormones that where significantly regulated by the meal were detected by a one-way repeated-measures ANOVA test where *** = P<0.001, * = P<0.05, and NS is not significant.

### Detailed Description

The present invention relates to methods for the extraction and separation of proteins with a molecular weight under 15 kDa from a biological sample chosen from plasma or serum. The separated fraction may then be used for detection and quantitation of proteins in downstream analysis within a short timescale. The method of the invention in its general sense is defined by claim 1. Also provided but not included in the claimed scope are proteins with a molecular weight under 15 kDa obtained or obtainable by the methods of the invention.

The present invention provides methods for the extraction and separation of proteins with a molecular weight under 15 kDa from a biological sample, the methods comprising the steps of: dissociation of proteins with a molecular weight under 15 kDa from larger proteins in a biological sample, precipitation of larger proteins, and further separation of proteins based on size. The methods provide a separated fraction containing only proteins with a molecular weight under 15 kDa, which may be used for detection and quantitation in downstream analysis.

In some embodiments, the proteins dissociated and separated from larger proteins have a molecular weight under 15 kDa, under 14 kDa, under 13 kDa, under 13 kDa, under 12 kDa, under 11 kDa, under 10 kDa, under 9 kDa, under 8 kDa, under 7 kDa, under 6 kDa, under 5 kDa, under 4 kDa, under 3 kDa, under 2kDa, under 1 kDa, above 1 kDa or above 0.5kDa.

In some embodiments, the proteins dissociated and separated from larger proteins are present in biological samples at concentrations less than 1mg/mL, less than 900 µg/mL, less than 800 µg/mL, less than 700 µg/mL, less than 600 µg/mL, less than 500 µg/mL, less than 400 µg/mL, less than 300 µg/mL, less than 200 µg/mL, less than 100 µg/mL, less than 1 µg/mL, less than 900ng/mL, less than 800ng/mL, less than 700ng/mL, less than 600ng/mL, less than 500ng/mL, less than 400ng/mL, less than 300ng/mL, less than 200ng/mL, less than 100ng/mL, less than 1ng/mL, less than 900 pg/mL, less than 800 pg/mL, less than 700 pg/mL, less than 600 pg/mL, less than 500 pg/mL, less than 400 pg/mL, less than 300 pg/mL, less than 200 pg/mL, less than 100 pg/mL, less than 1 pg/mL, less than 900 fg/mL, less than 800 fg/mL, less than 700 fg/mL, less than 600 fg/mL, less than 500 fg/mL, less than 400 fg/mL, less than 300 fg/mL, less than 200 fg/mL, or less than 100 fg/mL.

### Protein-protein dissociation and protein precipitation

In some embodiments, protein-protein dissociation and protein precipitation is achieved by incubation of the biological sample in an extraction buffer to provide an extraction mixture. The extraction buffer may comprise HCl and ethanol. In some embodiments, the concentration of HCl in the extraction buffer is between 0.05M and 0.5M, between 0.1M and 0.4M, between 0.2M and 0.3M, or between 0.2M and 0.25M. In some embodiments, the HCl may be replaced by H₂SO₄, HNO₃, H₃PO₄ or a mixture of any two or more of these acids. In some embodiments, the concentration of ethanol in the extraction buffer is under 95% (v/v), under 90% (v/v), under 80% (v/v), under 70% (v/v), under 60% (v/v), under 50% (v/v). In some embodiments, the ethanol may be replaced by methanol, or 1-propanol, or 2-propanol, or a mixture of any two or more of these alcohols. In one embodiment, the extraction buffer comprises 0.25M HCl and 87.5% ethanol in H₂O. In some embodiments, the ratio of ethanol to 0.25M HCl in the extraction buffer is 9:1, 8:1, 7:1, 6:1, 5:1 or 4:1. In one embodiment, the ratio of ethanol to 0.25M HCl in the extraction buffer is 7:1.

In some embodiments, the volume of biological sample in the extraction mixture is under 40%, under 30%, under 20%, under 10%, or under 5% of the total volume of the incubation. In one embodiment, the biological sample comprises 10% of the total volume of the incubation.

In some embodiments, biological samples are obtained and immediately incubated in the extraction buffer. In some embodiments, biological samples are refrigerated prior to incubation in the extraction buffer. In some embodiments, biological samples are frozen and must be thawed prior to incubation in the extraction buffer. A person skilled in the art would use a technique such as vortexing the biological sample as a matter of routine to ensure adequate mixing of the thawed biological sample prior to incubation.

In some embodiments, the biological sample is incubated in the extraction buffer at room temperature. In some embodiments, the biological sample is incubated in the extraction buffer for under 120 minutes, under 110 minutes, under 100 minutes, under 90 minutes, under 80 minutes, under 70 minutes, under 60 minutes, under 50 minutes, under 40 minutes, under 30 minutes, under 20 minutes, under 15 minutes, or under 10 minutes. In one embodiment, the biological sample is incubated in the extraction buffer for 30 minutes.

In some embodiments, precipitated proteins are pelleted by centrifugation. In one embodiment, precipitated proteins are pelleted by centrifugation at room temperature.

In some embodiments, either chloroform, or ethylacetate, or methyl tert-butyl ether, is added to the supernatant to ensure lipid removal prior to SEC-based separations.

In some embodiments, the supernatant is treated with either solid phase extraction, or supported liquid extraction, to ensure lipid removal prior to SEC-based separations. Solvents and/or chromatographic matrices (resins) may be used to facilitate neutral lipid and phospholipid removal to facilitate downstream processing. Supported liquid extraction is a sample preparation technique which was first used approximately 40 years ago. Persons skilled in the art would be aware that numerous variations to the conditions and reagents used with this technique may be made depending on the mixture to be separated. Solid phase extraction is another well-known sample preparation technique. See Walker and Mills 2002 Ann Clin Biochem 39:464-477 for a review of suitable materials and applications.

### Size exclusion chromatography

Size exclusion chromatography has been used since the 1950s for numerous applications and with a wide range of analytes (Lindqvist and Storgards 1955 Nature 175: 511-512). A person skilled in the art would recognise that many variations of size exclusion chromatography exist, which can be used in the methods of the present invention to extract and separate proteins of interest. In some embodiments, size exclusion chromatography is denaturing size exclusion chromatography. In claimed embodiments, a UHPLC system is used in combination with size exclusion chromatography. In some embodiments (not part of the claimed invention), field-flow fractionation, or ultrafiltration, or capillary electrophoresis, or a combination of any two or more of these methods is used instead of size exclusion chromatography.

In some embodiments, size exclusion chromatography uses columns of pore size under 500*Å*, under 300*Å*, under 200*Å*, or under 100*Å*. In one embodiment, size exclusion chromatography uses a column of pore size 130*Å*. In some embodiments, size exclusion chromatography uses columns of length under 300 mm, under 200 mm, or under 100 mm. In one embodiment, size exclusion chromatography uses a column of length 300 mm. In some embodiments, size exclusion chromatography uses columns of diameter under 30 mm, under 10 mm, or under 5 mm. In one embodiment, size exclusion chromatography uses a column of diameter 7.8 mm. In some embodiments, size exclusion chromatography uses columns with particles of diameter under 20 µm, under 10 µm, under 5 µm, or under 2 µm. In one embodiment, size exclusion chromatography uses a column with particles of diameter 2.7 µm*.*

In some embodiments, the SEC running buffer used comprises water, acetonitrile and TFA. In some embodiments, the concentration of acetonitrile in the SEC running buffer is under 50% (v/v), under 40% (v/v), under 30% (v/v), under 20% (v/v), or under 10% (v/v). In some embodiments, the acetonitrile may be replaced by methanol, ethanol, 1-propanol, or 2-propanol, or a mixture of any two or more of these solvents. In one embodiment, the concentration of acetonitrile in the SEC running buffer is 30% (v/v). In some embodiments, the concentration of TFA in the SEC running buffer is under 5% (v/v), under 1% (v/v), under 0.1% (v/v), or under 0.01% (v/v). In one embodiment, the concentration of TFA in the SEC running buffer is 0.1% (v/v). In some embodiments, the TFA may be replaced by formic acid, acetic acid, or heptafluorobutyric acid, or a mixture of any two or more of these acids.

In some embodiments, the fraction collected in size exclusion chromatography comprises or consists of proteins of molecular weight under 15 kDa, under 14 kDa, under 13 kDa, under 12 kDa, under 11 kDa, under 10 kDa, under 9 kDa, under 8 kDa, under 7 kDa, under 6 kDa, under 5 kDa, under 4 kDa, or under 3 kDa. In some embodiments, the fraction comprises or consists of proteins of molecular weight between 15 kDa and 2 kDa, between 14 kDa and 2 kDa, between 13 kDa and 2 kDa, between 12 kDa and 2 kDa, between 11 kDa and 2 kDa, between 10 kDa and 2 kDa, between 9 kDa and 2 kDa, between 8 kDa and 2 kDa, or between 7 kDa and 2 kDa. In some embodiments, the fraction comprises or consists of proteins of molecular weight between 15 kDa and 3 kDa, between 14 kDa and 3 kDa, between 13 kDa and 3 kDa, between 12 kDa and 3 kDa, between 11 kDa and 3 kDa, between 10 kDa and 3 kDa, between 9 kDa and 3 kDa, between 8 kDa and 3 kDa, or between 7 kDa and 3 kDa. In some embodiments, the fraction comprises or consists of proteins of molecular weight between 15 kDa and 4 kDa, between 14 kDa and 4 kDa, between 13 kDa and 4 kDa, between 12 kDa and 4 kDa, between 11 kDa and 4 kDa, between 10 kDa and 4 kDa, between 9 kDa and 4 kDa, between 8 kDa and 4 kDa, or between 7 kDa and 4 kDa. In some embodiments, the fraction comprises or consists of proteins of molecular weight between 15 kDa and 5 kDa, between 14 kDa and 5 kDa, between 13 kDa and 5 kDa, between 12 kDa and 5 kDa, between 11 kDa and 5 kDa, between 10 kDa and 5 kDa, between 9 kDa and 5 kDa, between 8 kDa and 5 kDa, or between 7 kDa and 5 kDa. In some embodiments, the fraction comprises or consists of proteins of molecular weight between 10 kDa and 2 kDa **(****Figure 1b****).** One advantage to the use of the fractions obtained by the methods of the present invention is the removal of small degradation products with a molecular weight of under 2 kDa.

### Analysis of Low Molecular Weight Protein Fractions

Proteins fractionated may be examined by many methods including: bottom-up mass spectrometry; top-down mass spectrometry; structural analysis of individual components using X-ray crystallography or NMR; affinity-reagent based assays (for example with aptamers or antibodies) such as ELISA, western blotting, immunoprecipitation, or proximity extension assay. The methods of the present invention involve analyzing the proteins by mass spectrometry. Other analytical techniques are disclosed but not claimed.

In one embodiment, the protein fraction is analysed by bottom-up mass spectrometry as described below.

### Disulfide bond reduction, alkylation and trypsin digestion

Proteins extracted using methods of the present invention frequently contain multiple disulfide bonds, for example, membrane proteins, or secreted proteins of clinical relevance such as insulin and hepcidin. In claimed embodiments, disulfide bonds are reduced in the collected fraction obtained by SEC, to avoid the introduction of intramolecular or intermolecular crosslinked peptides for downstream analysis by tandem mass spectrometry. In some embodiments, the reducing agent is TCEP. In some embodiments, the reducing agent is 2-mercaptoethanol. In some embodiments, the reducing agent is THPP. In some embodiments, the reducing agent is DTT.

In claimed embodiments, the reduced sulfhydryl groups are alkylated to prevent reformation of disulfide bonds. In some embodiments, the alkylating agent is iodoacetamide. In some embodiments, the alkylating agent is acrylamide. In some embodiments, the alkylating agent is N-ethylmaleimide. In some embodiments, the alkylating agent is 4-vinylpyridine. In some embodiments, the alkylating agent is iodoacetamide. In some embodiments, the alkylating agent is chloroacetamide. Disulfide bond reduction and alkylation are routine steps performed by the person skilled in the art to facilitate downstream identification of peptides during mass spectrometry, and many different reagents and conditions are commonly used (Suttapitugsakul et al. 2017 Molecular Biosystems 13(12):2574-2582).

In some embodiments, trypsin, or LysC, or a combination of trypsin and LysC is added to the samples to break down protein into fragments for downstream analysis. In some embodiments, trypsin is added at a ratio of 1:20 (µg trypsin to µg protein), or 1:50 (µg trypsin to µg protein), or 1:100 (µg trypsin to µg protein).

In some embodiments, a peptide clean-up step is performed using solid phase extraction following trypsin digestion and prior to downstream analysis. In some embodiments, peptides are bound to a strong cation exchange resin and eluted with a solution comprising ammonium hydroxide and acetonitrile.

In some embodiments, the sample is fractionated using reverse phase chromatography prior to downstream analysis. In some embodiments, high pH reverse phase chromatography is used. In some embodiments, peptides are resuspended in formic acid prior to high pH reverse phase chromatography. In some embodiments, buffers comprising acetonitrile and ammonium formate are used during fractionation.

### Mass Spectrometry

In claimed embodiments, samples are further analysed using mass spectrometry. In some embodiments, the mass spectrometer is a liquid chromatography-tandem mass spectrometer. In some embodiments, the mass spectrometer is a nano-liquid chromatography-tandem mass spectrometer. In some embodiments, the ionization technique is electrospray ionization and matrix-assisted laser desorption-ionization. In some embodiments, the ionization technique is nanospray electrospray ionization. In some embodiments, peptides are resolved over a gradient from 5% acetonitrile to 40% acetonitrile on a reversed-phase chromatography column. In some embodiments, peptides are resolved over a gradient from 5% acetonitrile to 60% acetonitrile on a reversed-phase chromatography column. In some embodiments, peptides are resolved over a gradient from 5% acetonitrile to 90% acetonitrile on a reversed-phase chromatography column. In some embodiments, peptides are resolved over a gradient from 2% acetonitrile to 100% acetonitrile on a reversed-phase chromatography column. In some embodiments, fragmentation is achieved via collision-induced dissociation, higher-energy collisional dissociation, electron capture dissociation, or ultraviolet photodissociation. In some embodiments, fragmentation is achieved via higher energy collisional dissociation.

In some embodiments, the data acquisition method is data-dependent acquisition. In other embodiments, the data acquisition method is data-independent acquisition. In some other embodiments, the data acquisition method is selected reaction monitoring (SRM), also commonly known as multiple reaction monitoring (MRM). In some embodiments, the data acquisition method is parallel reaction monitoring (PRM). The types of analysis that can be performed using mass spectrometry are well known in the art (Zhang et al. 2014 Current protocols in molecular biology 108:10.21.1-10.21.30).

### Exemplary Applications

In one embodiment, the methods of the present invention are used to create an assay which enriches small-protein hormones in a biological sample, enabling rapid, specific and sensitive quantitation. The assay is referred to herein as the small-protein hormone enrichment assay (sPHEA). By way of non-limiting example only, reference is made to **Figure 1a****,** which provides a representative schematic for the workflow of the sPHEA. In this example, blood plasma is contacted in an extraction buffer comprising hydrochloric acid and ethanol for protein-protein dissociation and protein removal. Lipids are then removed from the sample prior to denaturing SEC. Disulfide bonds are reduced in the collected fraction, followed by the steps of alkylation and trypsin digestion. A peptide clean-up step then follows, prior to analysis by LC-MS/MS.

In some embodiments, the methods are used to detect proteins in a biological sample. In some embodiments, the methods are also used for quantitation. In some embodiments, the methods are used for detection of a single protein. In some embodiments, the methods are also used for the quantitation of a single protein. In some embodiments, the methods are also used for the absolute quantitation of a single protein. In some embodiments, a stable-isotope labelled standard is used for absolute quantitation. In some embodiments, a stable-isotope labelled standard may be used for absolute quantitation of insulin in a biological sample. In one embodiment, a stable-isotope labelled standard is used for absolute quantitation using the sPHEA. By way of non-limiting example only, reference is made to **Figure 2a****,** which provides a representative schematic for a workflow of the use of the sPHEA with a stable-isotope labelled standard for absolute quantitation. In this example, stable-isotope (¹⁵N)-labelled intact human insulin was used to establish a standard curve using spike-in of the heavy analogue to raw plasma prior to the start of the sPHEA workflow. A targeted PRM-based LC-MS/MS analysis method was established that enabled combined quantitation of the light and heavy insulin beta chain peptide fragments in the same MS/MS (PRM) spectrum

In some embodiments, the methods are used for detection of a plurality of proteins. In some embodiments, the methods are also used for quantitation of a plurality of proteins.

The methods of the present invention may be used for the enrichment of low abundance small proteins in a biological sample for unbiased and sensitive quantitation in downstream analysis. The methods of the present invention may be used for untargeted proteome profiling of a biological sample. In some embodiments, untargeted profiling may detect active hormones/factors such as IGF-1, insulin, hepcidin, IGF-2, IL-36gamma, RANTES, chromogranin, SDF-1, granulins, defensins and guanylin. In some embodiments, untargeted profiling may detect fragments of larger proteins known to play a physiological role, including the C-terminus of alphal-antitrypsin (SERPINA1), or SPAAT, which regulates neutrophil extracellular trap (NET) formation. The methods of the present invention may be used to study numerous important hormones in a single rapid assay.

The methods of the present invention may be used to detect previously uncharacterized proteins. In some embodiments, the methods of the present invention may be used to detect novel hormones and biomarkers. In some embodiments, the methods of the present invention may be used to detect previously uncharacterized hormones using data dependent acquisitions. In some embodiments, this can be achieved within a short timescale.

In some embodiments, extensive fractionation of the trypsin-digested peptides prior to downstream analysis can identify over 900 proteins derived from over 6000 peptide precursors.

In some embodiments, the proteins are hormones. In some embodiments, the proteins are the clinically-relevant active chains of hormones. In some other embodiments, the proteins are cytokines. In some embodiments, the proteins are chemokines. In some embodiments, the proteins are enzymes. In some embodiments, the proteins are regulatory factors. In some embodiments, the proteins are carrier proteins. In some embodiments, the proteins are transport proteins.

Biological samples suitable for use with the present invention include any biological sample chosen from plasma or serum containing the analyte of interest. A sample could be obtained from any biological source, such as an animal, a cell culture, or an organ culture. Biological samples may include plasma or serum. In certain embodiments, samples are obtained from a mammal, such as a mouse, rat or primate. The mammalian animals may be humans. The samples could be obtained from a person presenting in a clinical setting for diagnosis, prognosis or treatment of a disease or condition.

In some embodiments, the methods of the present invention may be used to monitor the levels of proteins in biological samples over time. In some embodiments, the methods of the present invention may be used to monitor the levels of exogenous therapeutic proteins in biological samples over time. Exogenous peptides have been used as therapeutics since the 1920s (Lau and Dunn 2018 Bioorganic and Medicinal Chemistry 26: 2700-2707), In some embodiments, the methods of the present invention may be used to monitor the levels of commercial therapeutic insulins in biological samples over time. Many therapeutic insulins are largely modified in the beta-chain, and most have altered amino acid sequences compared to the endogenous hormone that can be differentiated by LC-MS/MS.

The methods of the present invention may be used for multiplexed small-protein hormone analysis of the vast number of human plasma samples collected during clinical trials. The methods of the present invention may find particular application in precision medicine approaches that require a global view of sample components, including small-protein hormones. In some embodiments, the methods of the present invention may be used for the quantitation of global changes during key immunological, metabolic and developmental perturbations. Purified peptides detected by the methods of the present invention may be examined by methods including top-down mass spectrometry, structural analysis of individual components, and different quantitative MS strategies to improve sample throughput such as isobaric tagging.

### Examples

The present invention will now be described with reference to the following specific examples, which should not be construed as in any way limiting.

### Example One: Detection of proteins over a wide dynamic range using the sPHEA

The sPHEA workflow involves the use of a combination of HCl and ethanol for protein-protein dissociation and protein removal by precipitation prior to denaturing high-resolution UHPLC-based SEC analysis. Disulfide bonds are then reduced in the collected fraction, followed by alkylation, trypsin digestion, peptide clean-up and LC-MS/MS analysis **(****Figure 1a****).**

This example demonstrates the ability of the sPHEA to solve the dynamic range problem for low abundance small-protein hormones with molecular weights <10 kDa, such as insulin. Human blood plasma samples were randomized using a random number generator prior to processing.

### 1.1 Chemicals and Reagents

Acetonitrile (Optima grade), water (Optima grade), ammonia, formic acid and isopropanol (Optima grade) were from Thermo Fisher Scientific (Massachusetts, USA). Proteomics-grade trypsin (Catalogue number T6567) and all other reagents were from Sigma Aldrich (Missouri, USA).

### 1.2 Dissociation-Precipitation-Lipid Removal

Plasma samples were thawed on ice and vortexed prior to aliquoting 50 µL into a 2 mL tube (Eppendorf) at room temperature that contained 450 µL of extraction buffer (0.25 M HCl, 87.5% ethanol in H₂O). Samples were vortexed every 15 min and incubated for a total of 30 min at room temperature. Precipitated proteins were pelleted by centrifugation at 8,400 x g for 10 min at room temperature. The supernatant was moved to a new 2 mL tube and 125 µL of chloroform added and the tube vortexed. This was a key step to ensure lipid removal prior to SEC-based separations. To facilitate phase separation, 500 µL of water was added and the tube vortexed prior to centrifugation for 10 minutes at 7,000 x g at room temperature. The clear top phase (~850 µL) was retained (peptide supernatant) while avoiding any pellet and the yellow chloroform phase. The small-protein hormone containing supernatant was filtered using 0.45 µm Ultrafree^{®}-MC HV centrifugal filter units (Merck Millipore - Massachusetts, USA) prior to moving the filtrate into vials for UHPLC-based SEC separation.

### 1.3 Denaturing Small-Protein Targeted SEC

To isolate proteins <10 kDa from larger proteins in the small-protein hormone containing supernatant a Dionex Ultimate 3000 Bio-RS UHPLC system (Thermo Fisher Scientific) was used combined with an Agilent (California, USA) AdvanceBio SEC column with 130 Å pores, 2.7 µm particles, dimensions of 7.8 x 300 mm. The column was equilibrated with 10 column volumes of denaturing SEC running buffer (30% acetonitrile and 0.1% TFA) prior to sample analysis. Standards consisting of either ubiquitin (Sigma - 79586-22-4), or a HPLC peptide standard mix (Sigma - H2016-1VL) diluted in SEC running buffer were used for mass calibration. The small-protein hormone containing supernatant was stored in the auto-sampler at 4°C prior to analysis and for each SEC separation, 200 µL was injected onto the column. Each SEC separation was performed for 25 min (1.5 column volumes) at a flow rate of 1 mL min⁻¹ at a column temperature of 30°C. The eluting proteins were monitored by UV absorbance at 215 and 280 nm. Only one fraction was collected into a low protein binding 2 mL tube or 2mL 96-deep-well plate (Eppendorf) between 6 min to 8 min retention time. This fraction corresponded to proteins of molecular weight between <10 kDa and >2 kDa, which had a total volume of 2 mL, and the fractions were stored at 4°C prior to subsequent processing.

### 1.4 Peptide Reduction, Alkylation and Trypsin Digestion

The collected fraction was dried using a GeneVac EZ-2, using the HPLC setting at 45°C and the dried proteins were resuspended in 100 µL of 50 mM triethylammonium bicarbonate (TEAB) pH 8.5 in H₂O. Disulfide bonds were reduced by addition of DTT to a final concentration of 5 mM and incubated on a thermomixer at 95°C at 1000 rpm for 10 min. To alkylate the reduced sulfhydryl groups chloroacetamide was added to a final concentration of 20 mM and samples incubated on a thermomixer at 95°C at 1000 rpm for 10 min. For trypsin digestion samples were cooled to room temperature and trypsin was added at a ratio of 1:20 (trypsin to protein), where each SEC fraction contained ~ 4 µg protein and therefore 200 ng was added. The samples were incubated for 16 h at 37°C at 500 rpm on a thermomixer to digest. To stop the digest 10% TFA in H₂O was added to achieve a 1% final concentration.

### 1.5 Peptide Clean- Up using SDB-RPS StageTips

SDB-RPS StageTips were generated by punching double-stacked SDB-RPS discs (Sigma, Cat#66886-U) with an 18-gauge needle and mounted in 200 µL tips (Eppendorf). For clean-up utilizing the Spin96, StageTips were inserted into a holder and placed in the top, which was then stacked onto the wash-bottom containing a polypropylene 96-well microtitre plate to collect the sample flow-through and washes. Each tip was wetted with 100 µL of 100% acetonitrile and centrifuged at 1,000 x g for 1 min. Following wetting, each StageTip was equilibrated with 30% methanol/1% TFA, followed by 100 µL of 0.1% TFA in H₂O, with centrifugation for each at 1,000 x g for 3 min. Each StageTip was then loaded with the equivalent of ~10 µg peptide in 1% TFA (<100 µL total volume per spin). The peptides were washed once with 100 µL of 0.2% TFA in water, which was followed by one wash with 100 µL of 99% isopropanol/1% TFA. For elution of peptides, the wash-bottom was exchanged with a bottom containing a holder supporting an unskirted PCR plate that has been trimmed to fit. To elute, 100 µL of 5% ammonium hydroxide/80% acetonitrile was added to each tip and centrifuged as above for 5 min. Samples in the PCR plate were dried using a GeneVac EZ-2 using the ammonia setting at 35°C for 1 h 15 m. Dried peptides were resuspended in 7 µL of 5% formic acid per 200 µL SEC injection of small-protein hormone-containing supernatant and stored at 4°C until analyzed by LC-MS.

### 1.6 High pH Reverse Phase Chromatography

Pooled trypsin digested plasma proteins processed using sPHEA (100 µg total) in 5% formic acid were subjected to high pH reverse phase chromatography on a Thermo Scientific Dionex Ultimate 3000 BioRS system with a fractionation auto-sampler, using a Waters XBridge Peptide BEH C18 column (130 Å, 3.5 µm, 4.6 mm X 250 mm, Cat No. 186003570). The column was incubated at 30°C with a constant flow rate of 1 mL/min, with buffer A containing 2% acetonitrile (ACN) and 10 mM ammonium formate (pH 9) and buffer B containing 80% ACN and 10 mM ammonium formate (pH 9). Fractions were collected every 8.75 s from a retention time of 2 min to 16 min (96 pseudo fractions, concatenated into 16 fractions total). Peptides were separated by a linear gradient from 10% to 40% buffer B for the first 11 min and 100% buffer B for the remaining time. The fractions were collected in a 2 mL protein low-bind 96-well deepwell plate (Eppendorf) across 16 wells in a concatenated pattern using tube wrapping.

### 1.7 LC-MS/MS Acquisition

Using a Thermo Fisher Dionex RSLCnano UHPLC, peptides in 5% (v/v) formic acid (injection volume 3 µL) were directly injected onto a 45 cm x 75 µm C18 (Dr. Maisch, Ammerbuch, Germany, 1.9 m) fused silica analytical column with a ~10 µm pulled tip, coupled online to a nanospray ESI source. Peptides were resolved over gradient from 5% acetonitrile to 40% acetonitrile over various gradient lengths ranging from 15 min to 140 min with a flow rate of 300 nL min-1. Peptides were ionized by electrospray ionization at 2.3 kV. Tandem mass spectrometry analysis was carried out on either a Q- Exactive HF, or HFX mass spectrometer (ThermoFisher) using HCD fragmentation.

### 1.8 DDA

The data-dependent acquisition method used in this example acquired MS/MS spectra of the top 10 most abundant ions at any one point during the gradient.

### 1.9 DIA

The data-independent acquisitions in this example were performed using variable isolation widths for different m/z ranges. Stepped normalized collision energy of 25 +/-10% was used for all DIA spectral acquisitions.

### 1.10 LC-MS/MS Data Analysis - MaxQuant Analysis

RAW data were analyzed using the quantitative proteomics software MaxQuant (version 1.5.7.0). This version of MaxQuant includes an integrated search engine, Andromeda. Peptide and protein level identification were both set to a false discovery rate of 1% using a target-decoy based strategy. The database supplied to the search engine for peptide identifications contained both the human UniProt database downloaded on the 30^{th} September 2017, containing 42,325 protein sequence entries and the MaxQuant contaminants database. Mass tolerance was set to 4.5 ppm for precursor ions and MS/MS mass tolerance was 20 ppm. Enzyme specificity was set to semi-specific N-ragged trypsin (cleavage C-terminal to Lys and Arg) with a maximum of 2 missed cleavages permitted for the main search and fully specific trypsin (cleavage C-terminal to Lys and Arg) for the first search. Deamidation of Asn and Gln, oxidation of Met, pyro-Glu (with peptide N-term Gln) and protein N-terminal acetylation were set as variable modifications. Carbamidomethyl on Cys was searched as a fixed modification. Maxquant output was processed and statistical tests performed using the R software package (version 3.4.3). Processed data was plotted using Tableau (version 10.0.2).

### 1.11 Results

The UV absorbance profile from denaturing high-resolution UHPLC-based SEC analysis highlighted the large amount of proteins >10 kDa remaining in the extract after the dissociation-precipitation procedure and the comparatively low levels of proteins below 10 kDa. The dissociation-precipitation procedure used in this example prior to SEC analysis enabled 25 min molecular weight separations that were optimized for proteins <10 kDa, but also >2 kDa to avoid small degradation products. The fraction between about 10 kDa and about 2 kDa (6-8 minutes) was collected for subsequent reduction, alkylation, trypsin digestion and LC-MS/MS analysis **(****Figure 1b****).**

Using only 2 hour single-shot LC-MS/MS data-dependent acquisitions from samples each derived from 6 µL of plasma, peptides were identified from a wide variety of active hormones/factors including IGF-1, insulin, hepcidin, IGF-2, IL-36gamma, RANTES, chromogranin, SDF-1, granulins, defensins and guanylin. Fragments of larger proteins known to play a physiological role were also observed, including the C-terminus of alpha-1 antitrypsin (SERPINA1), or SPAAT, which regulates neutrophil extracellular trap (NET) formation. Further extensive fractionation of the trypsin digested peptides prior to LC-MS/MS analysis identified >900 proteins derived from >6,000 peptide precursors. These data were compared to proteins identified previously in raw non-depleted human plasma fractionated similarly (Geyer et al. 2016 Cell Systems 2:185-195) (Figure 1c).

Alongside the >125 known active small-protein hormones/factors detected , peptides were observed from uncharacterized proteins that may also function as hormonal factors. These include the uncharacterized protein C5ORF46, which is a 7.2 kDa mammal-specific protein detected in this example for the first time in human plasma. Details of selected known active small-protein hormones/factors detected are provided in **Tables 1 and 2.**

**Table 1 - Names and properties of selected Small Protein Hormones/Factors of Interest Detected using the sPHEA Method**

| **Gene name** | **Uniprot Entry** | **Uniprot Protein name** | **Uniprot Secreted** | **Uniprot Propeptide** | **Uniprot Full Protein Mass (Da)** |
|---|---|---|---|---|---|
| ADM | P35318 | ADM (Adrenomedullin) | + | + | 20420 |
| AGT | P01019 | Angiotensinogen | + | - | 53154 |
| BDNF | P23560 | Brain-derived neurotrophic factor | + | + | 27818 |
| C5orf46 | Q6UWT4 | Uncharacterized protein C5orf46 | + | - | 9693 |
| CAMP | P49913 | Cathelicidin antimicrobial peptide | + | + | 19301 |
| CCL14 | Q16627 | C-C motif chemokine 14 | + | - | 10678 |
| CCL15 | Q16663 | C-C motif chemokine 15 | + | - | 12248 |
| CCL16 | O15467 | C-C motif chemokine 16 | + | - | 13600 |
| CCL18 | P55774 | C-C motif chemokine 18 | + | - | 9849 |
| CCL19 | Q99731 | C-C motif chemokine 19 | + | - | 10993 |
| CCL27 | Q9Y4X3 | C-C motif chemokine 27 | + | - | 12618 |
| CCL3 | P10147 | C-C motif chemokine 3 | + | - | 10085 |
| CCL5 | P13501 | C-C motif chemokine 5 | + | - | 9990 |
| CHGA | P10645 | Chromogranin-A | + | - | 50688 |
| CHGB | P05060 | Secretogranin-1 | + | - | 78276 |
| CXCL1 | P09341 | Growth-regulated alpha protein | + | - | 11301 |
| CXCL12 | P48061 | Stromal cell-derived factor 1 | + | - | 10666 |
| CXCL3 | P19876 | C-X-C motif chemokine 3 | + | - | 11342 |
| CXCL5 | P42830 | C-X-C motif chemokine 5 | + | - | 11972 |
| CYTL1 | Q9NRR1 | Cytokine-like protein 1 | + | - | 15577 |
| DCD | P81605 | Dermcidin | + | + | 11284 |
| DEFA1 | P59665 | Neutrophil defensin 1 | + | + | 10201 |
| DEFA6 | Q01524 | Defensin-6 | + | + | 10975 |
| DEFB1 | P60022 | Beta-defensin 1 | + | + | 7420 |
| GAL | P22466 | Galanin peptides | + | + | 13302 |
| GAST | P01350 | Gastrin | + | + | 11394 |
| GCG | P01275 | Glucagon | + | + | 20909 |
| GDF15 | Q99988 | Growth/differentiation factor 15 | + | + | 34140 |
| GHRL | Q9UBU3 | Appetite-regulating hormone | + | + | 12911 |
| GIP | P09681 | Gastric inhibitory polypeptide | + | + | 17108 |
| GRN | P28799 | Granulins | + | - | 63544 |
| GUCA2A | Q02747 | Guanylin | + | - | 12388 |
| GUCA2B | Q16661 | Guanylate cyclase activator 2B | + | + | 12069 |
| HAMP | P81172 | Hepcidin | + | + | 9408 |
| IGF1 | P05019 | Insulin-like growth factor I | + | + | 21841 |
| IGF2 | P01344 | Insulin-like growth factor II | + | + | 20140 |
| INHBB | P09529 | Inhibin beta B chain | + | + | 45122 |
| INHBC | P55103 | Inhibin beta C chain | + | + | 38238 |
| INHBE | P58166 | Inhibin beta E chain | + | + | 38561 |
| INS | P01308 | Insulin | + | + | 11981 |
| KISS1 | Q15726 | Metastasis-suppressor KiSS-1 | + | - | 14705 |
| KNG1 | P01042 | Kininogen-1 | + | - | 71957 |
| MIF | P14174 | Macrophage migration inhibitory factor | + | - | 12476 |
| MLN | P12872 | Promotilin | + | - | 12920 |
| MSTN | 014793 | Growth/differentiation factor 8 | + | + | 42750 |
| NPPA | PO1160 | Natriuretic peptides A | + | + | 16708 |
| NPPC | P23582 | C-type natriuretic peptide | + | + | 13246 |
| NTS | P30990 | Neurotensin/neuromedin N | + | - | 19795 |
| PCSK1N | Q9UHG2 | ProSAAS | + | - | 27372 |
| PDGFA | P04085 | Platelet-derived growth factor subunit A | + | + | 24043 |
| PDGFB | P01127 | Platelet-derived growth factor subunit B | + | + | 27283 |
| PNOC | Q13519 | Prepronociceptin | + | + | 20295 |
| RETN | Q9HD89 | Resistin | + | - | 11419 |
| SCG2 | P13521 | Secretogranin-2 | + | + | 70941 |
| SCG5 | P05408 | Neuroendocrine protein 7B2 | + | - | 23730 |
| SCGB1A1 | P11684 | Uteroglobin | + | - | 9994 |
| SERPINA1 | P01009 | Alpha-1-antitrypsin | + | - | 46737 |
| SLURP1 | P55000 | Secreted Ly-6/uPAR-related protein 1 | + | - | 11186 |
| SPINK1 | P00995 | Serine protease inhibitor Kazal-type 1 | + | - | 8507 |
| SPINK5 | Q9NQ38 | Serine protease inhibitor Kazal-type 5 | + | - | 120714 |
| SRGN | P10124 | Serglycin | + | - | 17652 |
| TAC3 | Q9UHF0 | Tachykinin-3 | + | + | 13438 |
| TFF1 | P04155 | Trefoil factor 1 | + | - | 9150 |
| TFF2 | Q03403 | Trefoil factor 2 | + | - | 14284 |
| TFF3 | Q07654 | Trefoil factor 3 | + | - | 10181 |
| TGFB1 | P01137 | Transforming growth factor beta-1 | + | - | 44341 |
| TNFRSF 12A | Q9NP84 | Tumor necrosis factor receptor superfamily member 12A | - | - | 13911 |
| WFDC12 | Q8WWY7 | WAP four-disulfide core domain protein 12 | + | - | 12050 |
| WFDC13 | Q8IUB5 | WAP four-disulfide core domain protein 13 | + | - | 10386 |

**Table 2 - Properties and sPHEA data for selected Small Protein Hormones/Factors of Interest Detected using the sPHEA Method**

| **Gene Name** | **Uniprot Smallest Processed Chain MW (Da)** | **Fractionated sPHEA Intensity** | **Fractionated Razor + Unique Peptides** |
|---|---|---|---|
| ADM | 2462 | 6.0E+08 | 3 |
| AGT | 552 | 5.6E+08 | 7 |
| BDNF | 13512 | 7.2E+07 | 1 |
| C5orf46 | 7216 | 6.3E+07 | 1 |
| CAMP | 4493 | 4.3E+08 | 4 |
| CCL14 | 7801 | 2.8E+10 | 10 |
| CCL15 | 6928 | 3.6E+09 | 12 |
| CCL16 | 11202 | 6.8E+08 | 2 |
| CCL18 | 7557 | 3.4E+10 | 11 |
| CCL19 | 8800 | 1.8E+07 | 1 |
| CCL27 | 10150 | 1.1E+08 | 2 |
| CCL3 | 7445 | 1.5E+07 | 1 |
| CCL5 | 7504 | 8.6E+09 | 11 |
| CHGA | 978 | 1.3E+09 | 9 |
| CHGB | 1342 | 5.4E+08 | 8 |
| CXCL1 | 7436 | 1.3E+07 | 1 |
| CXCL12 | 7610 | 1.4E+09 | 3 |
| CXCL3 | 7509 | 1.1E+09 | 6 |
| CXCL5 | 7706 | 3.5E+08 | 4 |
| CYTL1 | 13371 | 4.0E+09 | 6 |
| DCD | 2971 | 1.5E+10 | 16 |
| DEFA1 | 3377 | 2.1E+10 | 5 |
| DEFA6 | 3714 | 2.8E+07 | 4 |
| DEFB1 | 3935 | 3.1E+09 | 7 |
| GAL | 3157 | 9.1E+08 | 4 |
| GAST | 818 | 4.2E+07 | 1 |
| GCG | 3299 | 3.2E+08 | 6 |
| GDF15 | 12514 | 4.5E+06 | 1 |
| GHRL | 2548 | 7.5E+07 | 2 |
| GIP | 4984 | 2.9E+09 | 10 |
| GRN | 3149 | 4.0E+10 | 25 |
| GUCA2A | 1463 | 7.1E+09 | 8 |
| GUCA2B | 1672 | 2.8E+08 | 2 |
| HAMP | 2200 | 8.0E+09 | 8 |
| IGF1 | 7655 | 1.1E+11 | 21 |
| IGF2 | 4030 | 1.5E+11 | 27 |
| INHBB | 12817 | 7.0E+07 | 1 |
| INHBC | 12534 | 7.6E+07 | 2 |
| INHBE | 12465 | 7.8E+07 | 1 |
| INS | 2384 | 4.7E+08 | 2 |
| KISS1 | 1303 | 1.7E+08 | 4 |
| KNG1 | 528 | 2.1E+09 | 4 |
| MIF | N/A | 8.8E+07 | 2 |
| MLN | 2699 | 1.7E+07 | 1 |
| MSTN | 12407 | 1.4E+07 | 2 |
| NPPA | 3082 | 7.2E+08 | 5 |
| NPPC | 2200 | 1.2E+07 | 1 |
| NTS | 618 | 2.4E+08 | 6 |
| PCSK1N | 796 | 4.5E+07 | 2 |
| PDGFA | 14306 | 5.4E+08 | 3 |
| PDGFB | 12294 | 5.4E+08 | 3 |
| PNOC | 1809 | 2.4E+08 | 2 |
| RETN | 9554 | 1.5E+07 | 1 |
| SCG2 | 3679 | 5.9E+08 | 5 |
| SCG5 | 1502 | 3.8E+06 | 1 |
| SCGB1A1 | 7915 | 2.4E+09 | 6 |
| SERPINA1 | 5068 | 5.5E+11 | 108 |
| SLURP1 | 8853 | 3.0E+09 | 9 |
| SPINK1 | 6247 | 1.4E+08 | 1 |
| SPINK5 | 6482 | 6.7E+09 | 25 |
| SRGN | 14712 | 3.6E+09 | 4 |
| TAC3 | 1211 | 6.4E+08 | 6 |
| TFF1 | 6674 | 2.4E+08 | 2 |
| TFF2 | 11989 | 8.8E+07 | 1 |
| TFF3 | 6580 | 7.2E+08 | 4 |
| TGFB1 | 12795 | 4.3E+07 | 1 |
| TNFRSF 12A | 10925 | 7.4E+07 | 3 |
| WFDC12 | 9716 | 6.3E+07 | 2 |
| WFDC13 | 7978 | 2.5E+09 | 3 |

This example highlights the depth of protein detection achieved by the sPHEA based upon comparison with the PaxDB-based abundance estimates for >4,300 human plasma proteins, and shows that while extensive fractionation of either the sPHEA sample or the non-depleted sample allows detection of proteins over a wide dynamic range down to sub-ppb levels, insulin and many other small-protein hormones were only detected in the sPHEA method **(****Figure 1c****).** This example demonstrates rapid, specific and sensitive quantitation of >100 low abundance small-protein hormones (e.g. insulin, hepcidin, chemokines) in human plasma and importantly, the observation of the active chains of these hormones and not just the pro-peptides often characterised in previous studies. This example highlights the ability of the sPHEA to study numerous important hormones in a single rapid assay, greatly increasing feasibility while reducing cost, and highlights opportunities for discovery of new hormones and biomarkers produced by a variety of tissues/conditions.

### Example Two: Absolute quantification of insulin using heavy isotope labelling

In this example, the sPHEA workflow was used for absolute quantification of insulin using a stable-isotope-labelled standard. Insulin is a low-abundance hormone, occurring in plasma at <10 ng/mL. Using stable-isotope (¹⁵N)-labelled intact human insulin, a standard curve was established using spike-in of the heavy analogue to raw plasma prior to the start of the sPHEA workflow **(****Figure 2a****).** A targeted PRM-based LC-MS/MS analysis method was established that enabled combined quantification of the light and heavy insulin beta chain peptide fragments in the same MS/MS (PRM) spectrum (Figure 2b).

The peptide chosen for PRM analysis was unique to the beta-chain of insulin. For quantification, the intensities of multiple y and b fragment ions were used to generate a total intensity value for either the light, or heavy insulin.

### 2.1 ¹⁵N-Insulin Spike-in

Heavy human insulin was purchased from Sigma (SILuTMProt Insulin) labelled with ¹⁵N (>97% incorporation efficiency), which was recombinantly expressed in *Pichia pastoris.* The entire vial was resuspended in 2% acetic acid to a final concentration of 1.722 µM and the stock aliquoted into 0.5 mL low protein binding tubes for storage at -80°C. For generation of the standard curve, an intermediate dilution of 312.5 nM heavy insulin was made with 2% acetic acid in low protein binding tubes, which was spiked into plasma with a 10-fold dilution to make the most concentrated heavy insulin spike-in (45 µL plasma, 5 µL heavy insulin). For all other points on the standard curve 5-fold dilutions of the 312.5 nM intermediate stock were made with 2% acetic acid in low protein binding tubes, prior to being spiked into plasma with a 10-fold dilution. These 50 µL samples were then used for the sPHEA.

### 2.2 Parallel Reaction Monitoring

The Parallel Reaction Monitoring (PRM) method used in this example was a tier 3 analysis. This PRM method used a 15 min gradient separation and acquired targeted MS/MS spectra for the +4 charge-state tryptic peptide derived from human insulin beta-chain (FVNQHLCGSHLVEALYLVCGER) as it was the most abundant precursor, which was carbamidomethylated at both cysteine residues. Both the light (m/z = 651.3227) and heavy ¹⁵N-labelled (m/z = 659.0496) forms of this peptide were co-isolated (MSX count = 2) using the same injection time (MSX isochronous ITs = ON). The isolation window was tested across numerous runs with windows between 0.4-1.2 Th and 0.7 Th was determined as the optimal isolation window. The normalized collision energy (NCE) was tested for energies between 17 and 35, with 19 being the optimum used due to the maximal abundance of large (>800 m/z) fragment ions. Background interference was determined by comparison to blank runs. All spectra were acquired at 60,000 resolution in profile mode and a maximum injection time for MS/MS (PRM) of 100 ms.

### 2.3 LC-MS/MS Data Analysis - PRM Data Analysis using Spectronaut Pulsar X

RAW data from the heavy insulin spike-in standard curve were analysed using the quantitative proteomics software Spectronaut Pulsar X (version 12.0.20491.11.25225 (Jocelyn)). The PRM data files were analysed using a "labelled" plasma library generated in house from the fractionated plasma small-protein hormone analysis. For each ion (either light, or heavy), the 5 most abundant fragment ions were selected for quantification and their intensity was summed. To quantify both the ¹⁵N heavy and light forms of insulin the "labelled" library was generated using the label modifications ¹⁵N (A, C, D, E, F, G, I, L, M, P, S, T, V, Y), ¹⁵N₂ (K, N, Q, W), ¹⁵N₃ (H) & ¹⁵N₄ (R). The workflow was set to use iRT profiling. All other settings were factory default.

### 2.4 Results

A targeted PRM-based LC-MS/MS analysis method was established in this example that enabled combined quantification of the light and heavy insulin beta chain peptide fragments in the same MS/MS (PRM) spectrum **(****Figure 2b****).** The peptide chosen for PRM analysis was unique to the beta-chain of insulin. For quantification, the intensities of multiple y and b fragment ions were used to generate a total intensity value for either the light, or heavy insulin.

The standard curve generated covered a concentration range spanning 3 orders of magnitude and showed excellent linearity and low CV values even at the lower concentrations **(****Figure 2c****).** Given that normal plasma insulin abundance after a meal is ~1 nM and in the fasted state is <0.2 nM, the standard curve established in this example is well suited to analysis over this physiological range.

This example demonstrates accurate absolute quantitation of the low abundance hormone insulin, and that the beta-chain of insulin can easily be monitored through its tryptic peptides. Consequently, this example highlights the possibility of using the sPHEA to monitor the plasma abundance of many commercial therapeutic insulins.

### Example Three: sPHEA analysis of a human intermittent fasting clinical trial

Intermittent fasting (IF) is a dietary intervention that is of significant interest due to the beneficial effects on metabolic health. However, very few studies have examined in an unbiased way the physiological changes induced by IF. In this example, the sPHEA workflow was used to examine the plasma hormone response across a longitudinal clinical trial of IF in humans.

The PREFER randomized controlled trial was a discovery-based, single-centre study in Adelaide, South Australia and was registered with Clinicaltrials.gov (NCT01769976). The Royal Adelaide Hospital Research Ethics Committee approved the study protocol, and all participants provided written, informed consent prior to their inclusion. Each subject was assigned a number allowing for de-identification. A total of 88 women were enrolled in the study of which 25 were assigned to the intermittent fasting with weight maintenance group (IF100) analyzed in this example, 3 participants withdrew during the diet period (2 due to time, 1 no longer wished to participate). The resulting 44 paired plasma samples were subjected to MS analysis with two samples from two different patients failing QC, leaving 40 patient samples for data analysis. Inclusion criteria were: aged 35-70 years; BMI 25-42 kg/m² ; weight-stable (within 5% of their screening weight) for >6 months prior to study entry; no diagnosis of type 1 or type 2 diabetes; non-smoker; sedentary or lightly active (i.e., <2 moderate to high-intensity exercise sessions per week); consumed <140 g alcohol/week; no personal history of cardiovascular disease, no diagnosis of eating disorders or major psychiatric disorders (including those taking antidepressants); ); not pregnant or breastfeeding; and not taking medication that may affect study outcomes (e.g., phentermine, orlistat, metformin, excluding antihypertensive/lipid lowering medication). The active trial period was 10 weeks, comprised of a 2-week lead-in period, and 8 weeks of dietary intervention.

During the lead-in period, participants consumed their normal diet and maintained their weight. Following this, participants were placed on an IF diet at 100% of calculated baseline energy requirements per week (i.e., weight maintenance). Energy requirements were calculated using an average of published equations, both of which use age, gender, height and weight variables. Due to the nature of the intervention, blinding was not possible.

### 3.1 Diet

On fed days, participants were provided with food equal to ~145% of energy requirements. On fasting days, participants consumed breakfast before 8 am (~37% of energy requirements were given at breakfast on fasting days) and were then instructed to "fast" for 24 h, until 8 am the following day. Participants were advised to fast on 3 nonconsecutive week-days per week. During the fasting period participants were allowed to consume water and limited amounts of energy-free foods (e.g., "diet" drinks, chewing gum, mints), black coffee and/or tea, and were provided with 250 mL of a very low energy broth (86 kJ/250 mL, 2.0 g protein, 0.1 g fat, 3.0 g carbohydrate) for either lunch, or dinner. Participants were free-living, and foods were provided by fortnightly delivery to their home, except for fresh fruits and vegetables. Portions of fruits and vegetables were standardized and participants allowed to self-select according to the number of serves specified in their individual menus (~10% overall energy intake).

### 3.2 Blood Collection and Analysis

Blood samples were collected directly into purple K2-EDTA vacutainers (Becton Dickinson) and placed on ice immediately after collection. Samples were centrifuged within 10 min of collection at 4°C and the plasma frozen at -80°C in cryotubes. Each sample was subject to <3 freeze-thaw cycles on ice.

### 3.3 Human plasma for sPHEA Setup

Human plasma was obtained from 5 healthy volunteers using 4mL purple K2-EDTA vacutainers (Becton Dickinson). Samples were immediately placed on ice and spun <15 min post-collection at 1,500 g for 15 min at 4°C. Blood samples were frozen at -80°C and exposed to <3 freeze-thaw cycles. The Royal Prince Alfred Hospital Research Ethics Review Committee approved the study protocol (X17-0129 & HREC/17/RPAH/183), and all participants provided written, informed consent prior to their inclusion.

### 3.4 Insulin Standard Curve

From previous Spin96 optimizations, three technical replicates of each sample were considered enough to account for experimental variation and provide statistically significant results.

### 3.5 LC-MS/MS Data Analysis - Spectronaut Pulsar X directDIA Analysis

RAW data were analyzed using the quantitative proteomics software Spectronaut Pulsar X (version 12.0.20491.11.25225 (Jocelyn)). The database supplied to the search engine for peptide identifications was a focused database generated in house from the fractionated plasma small-protein hormone analysis. Enzyme specificity was set to semi-specific N-ragged trypsin (cleavage C-terminal to Lys and Arg) with a maximum of 2 missed cleavages permitted. Deamidation of Asn and Gln, oxidation of Met, pyro-Glu (with peptide N-term Gln) and protein N-terminal acetylation were set as variable modifications. Carbamidomethyl on Cys was searched as a fixed modification. The workflow was set to use iRT profiling. The FDR was set to 1% using a target-decoy approach. Spectronaut generated a custom mass tolerance for each precursor ion. The threshold for accepting a precursor was set at a Q value <0.01 and each precursor must have >3 fragment ions. All other settings were factory default. Processed data was analyzed and statistical tests performed using the R software package (version 3.4.3) with plot generated using Tableau (version 10.0.2).

### 3.6 PREFER Trial Statistical Analysis

The number of participants was established from past studies, which suggested n = 22 per group would allow detection of a mean difference in glucose infusion rate (GIR) of 15 *µ*mol/kgFFM+17.7 between groups, with β = 0.8 and α = 0.05. A 10% drop out rate was allowed for, and thus a total of n = 25 per group were recruited. This example provides an analysis of the intermittent fasting with weight maintenance group (IF100), with plasma samples collected before and after an 8-week period of intermittent fasting. A Wilcox robust test was applied to these data to allow for proteins whose distribution for the difference between treatment groups across participants was not normally distributed. Specifically, Yuen's test was used on trimmed means for dependent (paired) samples. Fold changes comparing plasma peptide precursor abundance before and after intermittent fasting were calculated using the median. For plotting the variation in individual peptide precursor intensity measurements and clinical measures in response to IF, adjusted values were calculated that forced all participants to have the same mean value for each measure. For all datasets statistical analyses were performed using R (version 3.4.3) and processed data was plotted using Tableau (version 10.0.2). Data are shown as median ± 95% confidence interval, unless otherwise stated. Significance was set at P<0.05.

### 3.7 Hepcidin ELISA

Plasma hepcidin was measured by Human Hepcidin Quantikine ELISA Kit (R&D Systems, Minneapolis, USA; cat no. DHP250) according to the manufacturer's instructions. Briefly, 50 µL of assay diluent RD1-21 and 50 µL of either standards, or patient plasma (diluted 1:250 with calibrator diluent RD5-26), were added to assay wells and incubated for 2 h at room temperature. Following aspiration and 4 washes with wash buffer, 200 µL of human hepcidin conjugate were added to each well and incubated at room temperature for an additional 2 h at room temperature. Following 4 additional washes, 200 µL of substrate solution was added to all wells and allowed to develop over 30 min while protected from light, until colour had developed in all standard wells. 50 µL of stop solution was used to cease colour development and absorbance was determined at 450 nm with subtraction of a 540 nm reference on a Tecan M200 Pro plate reader (Tecan, Switzerland).

### 3.8 Results

The small-protein hormones from each of the participant plasma samples were quantified using a 2 h single shot LC-MS/MS analysis using a data-independent acquisition (DIA) approach. This analysis quantified >3400 peptide precursors, corresponding to >2000 unique peptides across >500 proteins. Using a paired test statistic, 235 peptide precursors were detected that were significantly changed (P<0.05) due to the IF intervention **(****Figure 3a****).** The small-protein hormone displaying the largest fold-change was hepcidin-25, which was down-regulated ~3-fold after the IF intervention **(****Figure 3b****).** To validate findings, MS-based quantitative data were correlated with immunoassay-based analysis of the same plasma samples, which were specific for either hepcidin (ELISA), or insulin (RIA). This showed good agreement between the methods used in this example and more traditional measurement approaches **(****Figure 3c****).** In addition, the known correlation between GLP-1 abundance and plasma insulin levels was also observed from the intermittent fasting LC-MS/MS dataset obtained using the sPHEA **(****Figure 3c****).**

This example demonstrates how the sPHEA can be used to characterize the response to intermittent fasting in a human clinical trial cohort. The sPHEA identified that hepcidin-25 was significantly down-regulated by >3-fold in humans undergoing IF for 8 weeks. Hepcidin-25 is a liver-derived hormone that controls systemic iron homeostasis by inhibiting iron absorption in the small intestine and inhibiting iron release from the liver. The observation of decreased hepcidin-25 following IF would suggest the participants were becoming iron deficient, potentially through either alterations in their food choice during the IF intervention, or the IF regime itself. For example, the liver plays a key role in fasting responses and nutritional homeostasis. Previous studies have observed a 2-fold decrease in plasma hepcidin-25 after a single bout of fasting followed by refeeding. However, no previous study has reported a change in hepcidin abundance after IF.

### Example Four: sPHEA analysis of a human clinical trial using a mixed meal test intervention and longitudinal plasma sampling

Mixed meal tests are a common intervention used in metabolic assessment of humans. The mixed meal combines approximately equal proportions of carbohydrates, proteins and fats. When the mixed meal is consumed in a short time period by a fasted human, it generates a temporal response in blood hormone levels as the food moves from the stomach and along the intestine being progressively digested and absorbed. Blood samples are taken in the fasted state before the meal and then at short (15-60 min) time intervals after the meal is consumed for up to 3-4 hours. No studies to date have examined, in an unbiased way, the blood hormonal changes induced by a mixed meal in humans. In this example, the sPHEA workflow was used to examine the plasma hormone response to a mixed meal in humans.

Importantly, the samples used in this analysis contained the protease inhibitor aprotinin, which has been commonly added to clinical trial blood samples after collection for the last 40 years, as it preserves labile peptide hormones like GLP-1 from digestion by the protease DPP4 (and other proteases) present in plasma. This presents a potential problem as aprotinin will inhibit trypsin, the main protease used in the downstream bottom-up LC-MS/MS proteomics analysis. In addition, aprotinin itself is a small protein, that could generate peptides and disrupt our LC-MS/MS analysis of the endogenous plasma peptide hormones.

This example also demonstrates the use of a 96-well solid phase extraction plate for the removal of lipids from the ethanol-HCl extracted plasma, which replaces the manual tube-based chloroform lipid removal by liquid-liquid extraction. This is a key advance for the ability to automate the sPHEA protocol on robotic systems, as liquid-liquid extractions with solvents like chloroform are not readily amenable to robotic liquid-handling systems. This innovation would enable a robotic system to consistently process thousands of human plasma samples using the sPHEA method that would otherwise not be possible.

The human randomized controlled trial was conducted according to the principles outlined in the declaration of Helsinki and the study protocol was approved by the St Vincent's Hospital Human Research Ethics Committee (Sydney, Australia). All participants provided written informed consent prior to study commencement. The study was registered at clinicaltrials.gov (NCT02501343). Participants were recruited between June 2015 and May 2016 from the general population by advertisements on noticeboards at the St Vincent's Hospital precinct (Sydney, Australia) and the University of New South Wales (Sydney, Australia), and via social media and email distribution lists. Prospective participants were screened via a telephone interview and if eligible, an in-person screening was performed by a physician (PK) at the Clinical Research Facility at the Garvan Institute of Medical Research (Sydney). Exclusion criteria included pregnancy and lactation, a personal history of diabetes, treatment with glucose-lowering medication, fasting blood glucose > 7.0 mmol/L (126 mg/dL) and/or HbAlc > 6.5% (69 mmol/mol), and cardiovascular, respiratory, or inflammatory disease. Individuals treated with antihypertensive medications and medications known to affect glucose homeostasis were excluded. Individuals with serum creatinine > 90 □mol/L and/or eGFR < 60 mL/min/1.73 m2 or liver enzymes equal to or over twice the normal upper limit, current smokers, or excessive alcohol consumers (>20 g/day or 40 g/day for women and men, respectively) were excluded, as were individuals with unstable body weight in the three months preceding the study.

### 4.1 Diet

Participants were asked not to exercise or drink alcohol for 48 h prior to the meal studies. To minimize variation between studies, participants were asked to follow a similar routine before the studies, with particular attention to the meal content on the night before the study. Participants reported to the Clinical Research Facility in the morning after an overnight fast, were placed on a hospital bed, and an 18-gauge intravenous cannula was inserted in the antecubital fossa. Baseline venous blood samples were taken at t=-30min before administration of the intervention. The study capsules were administered 15 min prior to ingestion of a meal consisting of two breakfast muffins (Sausage and Egg McMuffin, McDonald's Corporation^{®}, Sydney, Australia) and 250 mL apple juice (Golden Circle^{®}, Brisbane, Australia). Nutritional breakdown of the meal was calculated using FoodWorks 7 (Xyris, Spring Hill, QLD, Australia) based on the ingredients provided by manufacturers. The meal consisted of 3,567 kJ total energy, 42g protein, 39g fat (of which 18g where saturated fat), 79g carbohydrates (of which 31g where sugars), and 1.3g sodium. Participants were given 20 min to consume the meal, with the 3 h follow up commencing at the completion of the meal (time = 0). Participants remained in bed in a reclined position for the following 3 h, with blood samples drawn and blood pressure, arterial stiffness and the hunger/satiety scores assessed periodically.

### 4.2 Blood Collection and Analysis

Blood samples were collected directly into purple K2-EDTA vacutainers (Becton Dickinson) and aprotinin was added and mixed by inversion before being placed on ice immediately after collection. Samples were centrifuged within 10 min of collection at 4°C and the plasma frozen at -80°C in cryotubes. Each sample was subject to <3 freeze-thaw cycles on ice.

### 4.3 Plasma protein Dissociation-Precipitation and Solid Phase Extraction for Lipid Removal

Plasma samples were thawed on ice and vortexed prior to aliquoting 50 µL into a 2 mL tube (Eppendorf) at room temperature that contained 450 µL of extraction buffer (0.25 M HCl, 87.5% ethanol in H₂O). Samples were vortexed every 15 min and incubated for a total of 30 min at room temperature. Precipitated proteins were pelleted by centrifugation at 8,400 x g for 10 min at room temperature. The supernatant was moved to one well of an Agilent Captiva EMR Lipid solid phase extraction plate (40mg resin per well). The Captiva plate was positioned above a 2mL deep-well 96-well collection plate and centrifuged for 10 min at 1,000g at room temperature. The flow-through containing peptide hormones and no lipids was retained and moved into vials for UHPLC-based SEC separation as described above.

### 4.4 Peptide Reduction, Alkylation and LysC+Trypsin Digestion

The collected fraction was dried using a GeneVac EZ-2, using the HPLC setting at 45°C and the dried proteins were resuspended in 100 µL of 50 mM triethylammonium bicarbonate (TEAB) pH 8.5 in H₂O. Disulfide bonds were reduced by addition of DTT to a final concentration of 5 mM and incubated on a thermomixer at 95°C at 1000 rpm for 10 min. To alkylate the reduced sulfhydryl groups chloroacetamide was added to a final concentration of 20 mM and samples incubated on a thermomixer at 95°C at 1000 rpm for 10 min. For digestion of the aprotinin to protect the trypsin to be used subsequently, the samples were cooled to room temperature and LysC (Wako) was added at a ratio of 1:20 (LysC to protein), where each SEC fraction contained ~ 4 µg protein and therefore 200 ng was added. The samples were vortexed incubated for 1 h at 37°C at 1,000 rpm. Trypsin was then added at a ratio of 1:20 (trypsin to protein), where each SEC fraction contained ~ 4 µg protein and therefore 200 ng was added. The samples were incubated for 16 h at 37°C at 1,000 rpm on a thermomixer to digest. To stop the digest 10% TFA in H₂O was added to achieve a 1% final concentration. Peptides were purified as described above.

### 4.5 LC-MS/MS Data Analysis - Spectronaut Pulsar X directDIA Analysis

RAW data were analyzed using the quantitative proteomics software Spectronaut Pulsar X (version 12.0.20491.11.25225 (Jocelyn)). The database supplied to the search engine for peptide identifications was a focused database generated in house from the fractionated plasma small-protein hormone analysis. Enzyme specificity was set to semi-specific N-ragged trypsin (cleavage C-terminal to Lys and Arg) with a maximum of 2 missed cleavages permitted. Deamidation of Asn and Gln, oxidation of Met, pyro-Glu (with peptide N-term Gln) and protein N-terminal acetylation were set as variable modifications. Carbamidomethyl on Cys was searched as a fixed modification. The workflow was set to use iRT profiling. The FDR was set to 1% using a target-decoy approach. Spectronaut generated a custom mass tolerance for each precursor ion. The threshold for accepting a precursor was set at a Q value <0.01 and each precursor must have >3 fragment ions. All other settings were factory default.

### 4.6 Statistical Analysis

As a pilot study the plasma from 4 male participants was analysed. A one-way repeated measures ANOVA analysis was applied to determine if any peptide precursor showed a significant deviation at any timepoint during the mixed meal test. Statistical analyses were performed using R (version 3.4.3) and processed data was plotted using Tableau (version 10.0.2). Data are shown as median ± 1.5x the interquartile range. Significance was set at P<0.05.

### 4.7 Results

The first analysis was to determine the effect of the aprotinin on the SEC separation profile to determine if it co-eluted in the fraction collected containing peptide hormones. Comparison of plasma with or without aprotinin showed that the aprotinin did elute at 6.25-6.5min within the collection window used for sPHEA (6-8min) **Figure 4****.** Therefore, an extra enzyme was included in the digestion step, the endoproteinase LysC, which is resistant to the effects of aprotinin. If a LysC digest was performed prior to the addition of trypsin to the sample, this would digest the aprotinin protein and destroy its trypsin inhibitory activity.

To examine whether a 96-well solid phase extraction would be able to replace chloroform as the lipid removal step, a commercially available solution was utilized for the removal of lipids from samples from Agilent, the Captiva EMR Lipid 96-well plate. Normally this plate is used for neutral lipid and phospholipid removal from small molecule metabolite samples (e.g. glucose, amino acids, etc). The manufacturer's recommended solvent of choice for plasma extraction is 100% acetonitrile. Therefore, three different conditions were compared: 1) manual chloroform-based lipid removal from ethanol-HCl extracted plasma; 2) 96-well plate Captiva lipid removal from ethanol-HCl extracted plasma; and 3) 96-well plate Captiva lipid removal from 100% acetonitrile extracted plasma. These lipid-depleted fractions were then analysed by SEC **(****Figure 5****).** This showed that manual chloroform-based lipid removal and 96-well plate Captiva lipid removal from ethanol-HCl extracted plasma provided similar SEC profiles and similar levels of peptide/protein extraction. However, the use of 96-well plate Captiva lipid removal from 100% acetonitrile extracted plasma showed >100-fold reduction in protein/peptide extraction and was not considered further for the sPHEA protocol.

Using these two innovations described above, the small-protein hormones from each of the participant plasma samples were analyzed incorporating 96-well plate Captiva lipid removal from ethanol-HCl extracted plasma and combined LysC and trypsin digestion of the resulting SEC fractions. Peptides were quantified using a 2 h single shot LC-MS/MS analysis using a data-independent acquisition (DIA) approach as described above. Several positive and negative control hormones where examined for their temporal response to the mixed meal test **(****Figure 6****).** Two positive control hormones known to increase after a meal where insulin and gastric inhibitory peptide (GIP), which both had statistically significant peaks at 30min and 60min, respectively. In contrast, the negative control hormone hepcidin-25, which was not expected to change quickly after a meal had no significant response. One of the novel hormones detected previously, C5orf46, showed no significant response to the meal. Significant increased abundance was observed after 90min for the hormones granulin-3 (GRN) and insulin-like growth factor 1 (IGF1).

This example demonstrates how the sPHEA can be used in a high throughput and robotics compatible manner to characterize plasma samples containing commonly used blood plasma hormone preservatives such as aprotinin. These data represent the first use of unbiased blood plasma peptide hormone analysis to the response of humans to a meal.

## Claims

1. A method for separating proteins with a molecular weight under 15 kDa from plasma or serum, the method comprising the steps of:
contacting the plasma or serum in an extraction buffer comprising hydrochloric acid and ethanol to provide an extraction mixture;
separating the proteins with a molecular weight under 15 kDa from the extraction mixture by ultra high pressure liquid chromatography (UHPLC) and size exclusion chromatography;
further comprising the steps of:
(i) disulfide bond reduction of the proteins separated from the extraction mixture;
(ii) alkylation of reduced sulfhydryl groups in the proteins after step (i); and
(iii) contacting the proteins with a protease after step (ii),
prior to then analyzing the proteins by mass spectrometry.

2. The method according to claim 1, wherein the proteins have a molecular weight under 14 kDa, under 13 kDa, under 12 kDa, under 11 kDa, under 10 kDa, under 9 kDa, under 8 kDa, under 7 kDa, under 6 kDa, under 5 kDa, under 4 kDa, under 3 kDa, under 2 kDa, under 1 kDa, above 1kDa or above 0.5kDa.

3. The method according to claim 1 or claim 2, wherein the hydrochloric acid is present in the extraction buffer at a concentration of between 0.05M and 0.5M, between 0.1M and 0.4M, between 0.2M and 0.3M, or between 0.2M and 0.25M.

4. The method according to any one of claims 1 to 3, wherein the ethanol is present in the extraction buffer at a concentration of under 95% (v/v), under 90% (v/v), under 80% (v/v), under 70% (v/v), under 60% (v/v), or under 50% (v/v).

5. The method according to any one of claims 1 to 4, wherein the plasma or serum is present in the extraction mixture at a volume that is under 40%, under 30%, under 20%, under 10% or under 5% of the extraction buffer volume within the extraction mixture.

6. The method according to any one of claims 1 to 5, wherein the size exclusion chromatography is denaturing size exclusion chromatography.

7. The method according to any one of claims 1 to 6, further comprising the use of solid phase extraction and/or supported liquid extraction to remove a lipid from said extraction mixture.

8. The method according to anyone of claims 1-7, wherein the protease used in step (iii) is lysC and/or trypsin.

9. The method according to any one of claims 1 to 7, wherein
- the disulfide bond reduction is performed with DTT; and/or
- the proteins are fractionated using high pH reverse phase chromatography following contacting the proteins with a protease, and prior to analyzing the proteins by mass spectrometry.

10. The method according to any one of claims 1 to 9, wherein the mass spectrometry is performed using a liquid chromatography-tandem mass spectrometer.

11. The method according to any one of claims 1 to 10, wherein
- said analysing the proteins by mass spectrometry comprises an ionization technique selected from electrospray ionization and matrix-assisted laser desorption-ionization; and/or
- said analysing the proteins by mass spectrometry comprises a data acquisition method selected from the group consisting of data-dependent acquisition, data-independent acquisition, selected reaction monitoring and parallel reaction monitoring.

12. The method according to any one of claims 1 to 11, wherein the mass spectrometry is used to quantitate a single protein from the plasma or serum.

13. The method according to any one of claims 1 to 12, wherein the proteins comprise peptide hormones.

14. The method according to any one of claims 1 to 13, wherein the proteins comprise or consist of insulin and/or hepcidin.

15. The method according to any one of claims 1 to 14, wherein the proteins with a molecular weight under 15kDa are present in the plasma or serum at a concentration of less than 1mg/mL, less than 500 µg/mL, less than 100 µg/mL, less than 1 µg/mL, less than 500ng/mL, less than 100ng/mL, less than 1ng/mL, less than 500 pg/mL, less than 100 pg/mL, less than 1 pg/mL, less than 500fg/mLor less than 100fg/mL.

## Patentansprüche

1. Verfahren zum Trennen von Proteinen mit einem Molekulargewicht von unter 15 kDa von Plasma oder Serum, wobei das Verfahren die folgenden Schritte umfasst:
Inkontaktbringen des Plasmas oder Serums in einem Extraktionspuffer, der Salzsäure und Ethanol umfasst, um ein Extraktionsgemisch bereitzustellen;
Trennen des Proteins mit einem Molekulargewicht von unter 15 kDa von dem Extraktionsgemisch durch Ultrahochdruckflüssigkeitschromatographie (UHPLC) Größenausschlusschromatographie;
ferner umfassend die folgenden Schritte:
(i) Disulfidbindungsreduktion der Proteine, die von dem Extraktionsgemisch getrennt wurden;
(ii) Alkylierung von reduzierten Sulfhydrylgruppen in den Proteinen nach Schritt (i); und
(iii) Inkontaktbringen der Proteine mit einer Protease nach Schritt (ii) vor dem Analysieren der Proteine durch Massenspektrometrie.

2. Verfahren nach Anspruch 1, wobei die Proteine ein Molekulargewicht von unter 14 kDa, unter 13 kDa, unter 12 kDa, unter 11 kDa, unter 10 kDa, unter 9 kDa, unter 8 kDa, unter 7 kDa, unter 6 kDa, unter 5 kDa, unter 4 kDa, unter 3 kDa, unter 2 kDa, unter 1 kDa, über 1 kDa oder über 0,5 kDa aufweisen.

3. Verfahren nach Anspruch 1 oder Anspruch 2, wobei die Salzsäure in dem Extraktionspuffer in einer Konzentration von zwischen 0,05 M und 0,5 M, zwischen 0,1 M und 0,4 M, zwischen 0,2 M und 0,3 M oder zwischen 0,2 M und 0,25 M vorhanden ist.

4. Verfahren nach einem der Ansprüche 1 bis 3, wobei das Ethanol in dem Extraktionspuffer in einer Konzentration von unter 95 Vol.%, unter 90 Vol.%, unter 80 Vol.%, unter 70 Vol.%, unter 60 Vol.% oder unter 50 Vol.% vorhanden ist.

5. Verfahren nach einem der Ansprüche 1 bis 4, wobei das Plasma oder Serum in dem Extraktionsgemisch in einem Volumen vorhanden ist, das unter 40 %, unter 30 %, unter 20 %, unter 10 % oder unter 5 % des Extraktionspuffervolumens innerhalb des Extraktionsgemisches liegt.

6. Verfahren nach einem der Ansprüche 1 bis 5, wobei die Größenausschlusschromatographie eine Denaturierungsgrößenausschusschromatographie ist.

7. Verfahren nach einem der Ansprüche 1 bis 6, ferner umfassend die Verwendung von Festphasenextraktion und/oder unterstützter Flüssigextraktion, um ein Lipid aus dem Extraktionsgemisch zu entfernen.

8. Verfahren nach einem der Ansprüche 1-7, wobei die Protease, die in Schritt (iii) verwendet wird, Lys-C und/oder Trypsin ist.

9. Verfahren nach einem der Ansprüche 1 bis 7, wobei
- die Disulfidbindungsreduktion mit DTT durchgeführt wird; und/oder
- die Proteine unter Verwendung von Umkehrphasenchromatographie mit hohem pH-Wert nach dem Inkontaktbringen der Proteine mit einer Protease und vor dem Analysieren der Proteine durch Massenspektrometrie fraktioniert werden.

10. Verfahren nach einem der Ansprüche 1 bis 9, wobei die Massenspektrometrie unter Verwendung eines Massenspektrometers mit Flüssigchromatographiekopplung durchgeführt wird.

11. Verfahren nach einem der Ansprüche 1 bis 10, wobei
- das Analysieren der Proteine durch Massenspektrometrie eine Ionisierungstechnik umfasst, die aus Elektrospray-Ionisierung und Matrix-unterstützter Laser-Desorption/Ionisation ausgewählt ist; und/oder
- das Analysieren der Proteine durch Massenspektrometrie ein Datenerfassungsverfahren umfasst, das aus der Gruppe ausgewählt ist, die aus datenabhängiger Erfassung, datenunabhängiger Erfassung, ausgewählter Reaktionsüberwachung und paralleler Reaktionsüberwachung besteht.

12. Verfahren nach einem der Ansprüche 1 bis 11, wobei die Massenspektrometrie verwendet wird, um ein einzelnes Protein von dem Plasma oder Serum zu quantifizieren.

13. Verfahren nach einem der Ansprüche 1 bis 12, wobei die Proteine Peptidhormone umfassen.

14. Verfahren nach einem der Ansprüche 1 bis 13, wobei die Proteine Insulin und/oder Hepcidin umfassen oder aus diesen/diesem bestehen.

15. Verfahren nach einem der Ansprüche 1 bis 14, wobei die Proteine mit einem Molekulargewicht von unter 15 kDa in dem Plasma oder Serum in einer Konzentration von Folgendem vorhanden sind: weniger als 1 mg/ml, weniger als 500 µg/ml, weniger als 100 µg/ml, weniger als 1 µg/ml, weniger als 500 ng/ml, weniger als 100 ng/ml, weniger als 1 ng/ml, weniger als 500 pg/ml, weniger als 100 pg/ml, weniger als 1 pg/ml, weniger als 500 fg/ml oder weniger als 100 fg/ml.

## Revendications

1. Procédé pour séparer des protéines ayant un poids moléculaire inférieur à 15 kDa provenant de plasma ou de sérum, le procédé comprenant les étapes consistant à :
mettre en contact le plasma ou le sérum dans un tampon d'extraction comprenant de l'acide chlorhydrique et de l'éthanol pour fournir un mélange d'extraction ;
séparer les protéines ayant un poids moléculaire inférieur à 15 kDa à partir du mélange d'extraction par chromatographie liquide à ultra haute pression (UHPLC) et chromatographie d'exclusion stérique ;
comprenant en outre les étapes consistant à :
(i) réduire les ponts disulfure des protéines séparées à partir du mélange d'extraction ;
(ii) alkyler les groupes sulfhydryles réduits dans les protéines après l'étape (i) ; et
(iii)mettre en contact les protéines avec une protéase après l'étape (ii), avant d'analyser ensuite les protéines par spectrométrie de masse.

2. Procédé selon la revendication 1, dans lequel les protéines ont un poids moléculaire inférieur à 14 kDa, inférieur à 13 kDa, inférieur à 12 kDa, inférieur à 11 kDa, inférieur à 10 kDa, inférieur à 9 kDa, inférieur à 8 kDa, inférieur à 7 kDa, inférieur à 6 kDa, inférieur à 5 kDa, inférieur à 4 kDa, inférieur à 3 kDa, inférieur à 2 kDa, inférieur à 1 kDa, supérieur à 1 kDa ou supérieur à 0,5 kDa.

3. Procédé selon la revendication 1 ou la revendication 2, dans lequel l'acide chlorhydrique est présent dans le tampon d'extraction à une concentration comprise entre 0,05 M et 0,5 M, entre 0,1 M et 0,4 M, entre 0,2 M et 0,3 M, ou entre 0,2 M et 0,25 M.

4. Procédé selon l'une quelconque des revendications 1 à 3, dans lequel l'éthanol est présent dans le tampon d'extraction à une concentration inférieure à 95 % (v/v), inférieure à 90 % (v/v), inférieure à 80 % (v/v), inférieure à 70 % (v/v), inférieure à 60 % (v/v), ou inférieure à 50 % (v/v).

5. Procédé selon l'une quelconque des revendications 1 à 4, dans lequel le plasma ou le sérum est présent dans le mélange d'extraction à un volume qui est inférieur à 40 %, inférieur à 30 %, inférieur à 20 %, inférieur à 10 % ou inférieur à 5 % du volume du tampon d'extraction au sein du mélange d'extraction.

6. Procédé selon l'une quelconque des revendications 1 à 5, dans lequel la chromatographie d'exclusion stérique est une chromatographie d'exclusion stérique dénaturante.

7. Procédé selon l'une quelconque des revendications 1 à 6, comprenant en outre l'utilisation d'une extraction en phase solide et/ou d'une extraction liquide supportée pour éliminer un lipide à partir dudit mélange d'extraction.

8. Procédé selon l'une quelconque des revendications 1 à 7, dans lequel la protéase utilisée à l'étape (iii) est la lysC et/ou la trypsine.

9. Procédé selon l'une quelconque des revendications 1 à 7, dans lequel
- la réduction des ponts disulfure est effectuée avec du DTT ; et/ou
- les protéines sont fractionnées en utilisant une chromatographie en phase inverse à pH élevé suite à la mise en contact des protéines avec une protéase, et avant l'analyse des protéines par spectrométrie de masse.

10. Procédé selon l'une quelconque des revendications 1 à 9, dans lequel la spectrométrie de masse est effectuée en utilisant un spectromètre de masse en tandem couplé à la chromatographie liquide.

11. Procédé selon l'une quelconque des revendications 1 à 10, dans lequel
- ladite analyse des protéines par spectrométrie de masse comprend une technique d'ionisation choisie parmi l'ionisation par électropulvérisation et l'ionisation-désorption laser assistée par matrice ; et/ou
- ladite analyse des protéines par spectrométrie de masse comprend une méthode d'acquisition de données choisie dans le groupe constitué par l'acquisition dépendante des données, l'acquisition indépendante des données, le suivi de réaction sélectionnée et le suivi de réactions en parallèle.

12. Procédé selon l'une quelconque des revendications 1 à 11, dans lequel la spectrométrie de masse est utilisée pour quantifier une seule protéine provenant du plasma ou du sérum.

13. Procédé selon l'une quelconque des revendications 1 à 12, dans lequel les protéines comprennent des hormones peptidiques.

14. Procédé selon l'une quelconque des revendications 1 à 13, dans lequel les protéines comprennent ou sont constituées par de l'insuline et/ou de l'hepcidine.

15. Procédé selon l'une quelconque des revendications 1 à 14, dans lequel les protéines ayant un poids moléculaire inférieur à 15 kDa sont présentes dans le plasma ou le sérum à une concentration inférieure à 1 mg/mL, inférieure à 500 µg/mL, inférieure à 100 µg/mL, inférieure à 1 µg/mL, inférieure à 500 ng/mL, inférieure à 100 ng/mL, inférieure à 1 ng/mL, inférieure à 500 pg/mL, inférieure à 100 pg/mL, inférieure à 1 pg/mL, inférieure à 500 fg/mL ou inférieure à 100 fg/mL.
